(19) Europäisches Patentamt / European Patent Office / Office européen des brevets

(11) **EP 3 325 972 B1**

(12) **EUROPEAN PATENT SPECIFICATION**

(45) Date of publication and mention of the grant of the patent:
**19.08.2020 Bulletin 2020/34**

(51) Int Cl.:
***G01N 33/574*** *(2006.01)*

(21) Application number: **16826916.5**

(22) Date of filing: **22.07.2016**

(86) International application number:
**PCT/AU2016/000260**

(87) International publication number:
**WO 2017/011855 (26.01.2017 Gazette 2017/04)**

(54) **BIOMARKER COMBINATIONS FOR PROSTATE DISEASE**

BIOMARKERKOMBINATIONEN FÜR PROSTATAERKRANKUNGEN

COMBINAISONS DE BIOMARQUEURS POUR UNE MALADIE DE LA PROSTATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.07.2015 AU 2015902919**

(43) Date of publication of application:
**30.05.2018 Bulletin 2018/22**

(73) Proprietor: **Minomic International Ltd.**
**Macquarie Park, NSW 2113 (AU)**

(72) Inventors:
• **WALSH, Bradley**
**Turramurra, NSW 2074 (AU)**
• **CAMPBELL, Douglas**
**Cremorne, NSW 2090 (AU)**
• **SOON, Julie**
**North Bondi, NSW 2026 (AU)**

(74) Representative: **Plasseraud IP**
**66, rue de la Chaussée d'Antin**
**75440 Paris Cedex 09 (FR)**

(56) References cited:
• **NEAL SHORE ET AL: "GLYPICAN-1 AS A BIOMARKER FOR PROSTATE CANCER", JOURNAL OF UROLOGY., vol. 193, no. 4, 17 May 2015 (2015-05-17), pages e496-e496, XP055541638, BALTIMORE, MD, US ISSN: 0022-5347, DOI: 10.1016/j.juro.2015.03.083**

• **SHAILENDRA DWIVEDI ET AL: "Diagnostic and Prognostic Significance of Prostate Specific Antigen and Serum Interleukin 18 and 10 in Patients with Locally Advanced Prostate Cancer: A Prospective Study", ASIAN PACIFIC JOURNAL OF CANCER PREVENTION J CANCER PREV, vol. 12, no. 12, 1 January 2011 (2011-01-01), pages 1843-1848, XP055541959,**

• **Michael Naughton ET AL: "SCATTER FACTOR-HEPATOCYTE GROWTH FACTOR ELEVATION IN THE SERUM OF PATIENTS WITH PROSTATE CANCER", The Journal of Urology, 1 January 2001 (2001-01-01), 15 January 2019 (2019-01-15), pages 1325-1328, XP055542094, United States DOI: 10.1016/S0022-5347(01)69893-8 Retrieved from the Internet: URL:https://www.jurology.com/doi/pdf/10.10 16/S0022-5347(01)69893-8 [retrieved on 2018-01-15]**

• **DOUGLAS H. CAMPBELL ET AL: "Detection of glypican-1 (GPC-1) expression in urine cell sediments in prostate cancer", PLOS ONE, vol. 13, no. 4, 19 April 2018 (2018-04-19) , page e0196017, XP055541562, DOI: 10.1371/journal.pone.0196017**

• **SHORE, N. ET AL.: 'PII-LBA3 GLYPICAN-1 AS A BIOMARKER FOR PROSTATE CANCER' JOURNAL OF UROLOGY vol. 193, no. 4, April 2015, pages E410 - E411, XP029152678**

• **SERRETTA, V. ET AL.: 'Pilot study on predictive value of plasmatic levels of 9 angiogenetic biomarkers in selection of patients candidate to prostate biopsy' UROLOGIA vol. 80, no. 4, 26 September 2013, pages 297 - 301, XP009508448 DOI: 10.5301/UROLOGIA.5000023**

**Description**

**Technical Field**

The present invention relates generally to the fields of immunology and medicine. More specifically, the present invention relates to the diagnosis of prostate disease and the identification of biomarkers in biological samples which can be used in the detection of prostate disease. The scope of protection is defined by the appended claims.

**Background**

Prostate cancer is the most frequently diagnosed visceral cancer and the second leading cause of cancer death in males. According to the National Cancer Institute's SEER program and the Centers for Disease Control's National Center for Health Statistics, 233,000 cases of prostate cancer arose in 2014 (14% of all new cancer cases) with 29,480 deaths (5% of all cancer deaths) (see SEER Cancer Statistics Factsheets: Prostate Cancer. National Cancer Institute. Bethesda, MD, http://seer.cancer.gov/statfacts/html/prost.html).

Commonly used screening tests for prostate cancer include digital rectal exam (DRE) and detection of prostate specific antigen (PSA) in blood. DRE is invasive and imprecise, and the prevalence of false negative (i.e. cancer undetected) and false positive (i.e. indication of cancer where none exists) results from PSA assays is well documented. For example, standard PSA tests conducted at the recommended 4.0 ng/ml cutoff, are 86% sensitive to cancer patients but only 33% specific, producing false positives in roughly 67% of non-cancer patients (Hoffman et al. BMC Fam Pract. 2002, 3:19). The diagnostic value of PSA for prostate cancer is also limited due to its lack of specificity between benign and cancerous conditions. Elevated levels of PSA are detectable in a large percentage of patients with benign prostatic hyperplasia (BPH) and prostatitis (25-86%) (Gao et al., 1997, Prostate 31: 264-281), as well as in other nonmalignant disorders, which significantly limits the diagnostic specificity of this marker. For example, elevations in serum PSA of between 4 to 10 ng/ml are observed in BPH, and even higher values are observed in prostatitis, particularly acute prostatitis.

Upon a positive diagnosis with DRE or PSA screening, confirmatory diagnostic tests include transrectal ultrasound, biopsy, and transrectal magnetic resonance imaging (MRI) biopsy. These techniques are invasive and cause significant discomfort to the subject under examination Shore et al., Journal of Urology, vol. 193, no. 4, 17 May 2015, pages e496-e496, discloses the MiCheck test applied to GPC-1, wherein GPC-1 can discriminate between cancer, BPH and healthy (abs).

A general need exists for convenient, reliable and accurate tests for diagnosing prostate disease. Preferably, the tests may have the capacity to discern between prostate cancer and non-cancerous forms of prostate disease (e.g. benign prostatic hyperplasia (BPH)).

**Summary of the Invention**

The present inventors have identified combinations of biomarkers effective for the detection of prostate disease. In particular, the biomarker combinations may be capable of discerning between cancerous and non-cancerous forms of prostate disease. Accordingly, the present invention relates to these biomarker combinations and assays that utilize them for the detection of prostate disease.

The present disclosure thus relates at least to the following series of numbered examples below:

Example 1a: A method for determining whether a subject has benign prostatic hyperplasia (BPH) from prostate cancer (CaP), comprising:

(a) generating a threshold value for discerning between BPH and prostate cancer by:

detecting at least two analytes in a series of biological samples obtained from a population of subjects having BPH and a population of subjects having CaP, to thereby obtain an analyte level for each said analyte in each said biological sample of the series;
combining the analyte levels in a manner that allows discrimination between the BPH and CaP samples; and selecting a threshold value from the combined analyte levels and using said threshold value as a basis to discriminate between BPH and CaP in the sample; and

(b) detecting said at least two analytes in a biological sample from a subject to thereby obtain an analyte level for each said analyte in the subject's biological sample; and
(c) applying a suitable algorithm and/or transformation to the individual or combined analyte levels obtained from the subject's biological sample to thereby generate a patient analyte value for comparison to the threshold value; and

(d) determining whether the subject has BPH or CaP by comparison of the patient analyte value to the threshold value,

wherein the at least two analytes comprise glypican-1 (GPC-1) and are selected from any one of the following analyte combinations:

GPC-1 and hepatocyte growth factor (HGF);
GPC-1 and epidermal growth factor (EGF);
GPC-1 and plasminogen activator inhibitor 1 (PAI-1);
GPC-1 and granulocyte colony stimulating factor (G-CSF);
GPC-1 and human Interleukin 18 (HuIL-18);
GPC-1 and platelet derived growth factor AB.BB (PDGFAB.BB);
GPC-1 and platelet derived growth factor BB (PDGFBB);
GPC-1 and soluble CD40 Ligand (sCD40L);
GPC-1 and human granulocyte macrophage colony stimulating factor (HuGM-CSF);
GPC-1 and interferon gamma (IFN$\gamma$); and
GPC-1 and follistatin.

Example 1b: A method for determining whether a subject has benign prostatic hyperplasia (BPH) or prostate cancer (CaP), comprising:

(a) generating a threshold value for discerning between BPH and prostate cancer by:

detecting at least two analytes in a series of biological samples obtained from a population of subjects having BPH and a population of subjects having CaP, to thereby obtain an analyte level for each said analyte in each said biological sample of the series;
combining the analyte levels in a manner that allows discrimination between the BPH and CaP samples; and
selecting a threshold value from the combined analyte levels and using said threshold value as a basis to discriminate between BPH and CaP in the sample; and

(b) detecting said at least two analytes in a biological sample from a subject to thereby obtain an analyte level for each said analyte in the subject's biological sample; and

(c) applying a suitable algorithm and/or transformation to the individual or combined analyte levels obtained from the subject's biological sample to thereby generate a patient analyte value for comparison to the threshold value; and

(d) determining whether the subject has BPH or CaP by comparison of the patient analyte value to the threshold value.

wherein the at least two analytes comprise glypican-1 (GPC-1) and are selected from any one of the following analyte combinations:

GPC-1 and hepatocyte growth factor (HGF);
GPC-1 and epidermal growth factor (EGF);
GPC-1 and plasminogen activator inhibitor 1 (PAI-1);
GPC-1 and granulocyte colony stimulating factor (G-CSF);
GPC-1 and human Interleukin 18 (HuIL-18);
GPC-1 and platelet derived growth factor AB.BB (PDGFAB.BB);
GPC-1 and platelet derived growth factor BB (PDGFBB);
GPC-1 and soluble CD40 Ligand (sCD40L);
GPC-1 and human granulocyte macrophage colony stimulating factor (HuGM-CSF);
GPC-1 and interferon gamma (IFN$\gamma$); and
GPC-1 and follistatin.

Example 1c: A method of generating a threshold value for use in discriminating between benign prostatic hyperplasia (BPH) and prostate cancer (CaP), comprising:

(a) detecting at least two analytes in a series of biological samples obtained from a population of subjects known to have BPH and a population of subjects known to have CaP, to thereby obtain an analyte level for each said analyte in each said biological sample of the series;

(b) combining the analyte levels in a manner that allows discrimination between the BPH and CaP samples; and

(c) selecting the threshold value from the combined analyte levels to serve as a basis to discriminate between BPH and CaP;

wherein the at least two analytes comprise glypican-1 (GPC-1) and are selected from any one of the following analyte combinations:

GPC-1 and hepatocyte growth factor (HGF);
GPC-1 and epidermal growth factor (EGF);
GPC-1 and plasminogen activator inhibitor 1 (PAI-1);
GPC-1 and granulocyte colony stimulating factor (G-CSF);
GPC-1 and human Interleukin 18 (HuIL-18);
GPC-1 and platelet derived growth factor AB.BB (PDGFAB.BB);
GPC-1 and platelet derived growth factor BB (PDGFBB);
GPC-1 and soluble CD40 Ligand (sCD40L);
GPC-1 and human granulocyte macrophage colony stimulating factor (HuGM-CSF);
GPC-1 and interferon gamma (IFNγ); and
GPC-1 and follistatin.

Example 2. The method according to embodiment 1, wherein the at least two analytes are selected from any one of the following analyte combinations:

GPC-1, HGF and PAI-1;
GPC-1, HGF and human fibroblast growth factor beta (HuFGFb);
GPC-1, HGF and EGF;
GPC-1, HGF and PDGFBB;
GPC-1, HGF and follistatin;
GPC-1, HGF and G-CSF;
GPC-1, HGF and human interleukin 8 (HuIL-8);
GPC-1, HGF and soluble CD40 ligand (sCD40L);
GPC-1, HGF and human cutaneous T-cell attracting cytokine (CTACK - also known as C-C motif ligand 27 or CCL27);
GPC-1, HGF and PDGFAB.BB;
GPC-1, HGF and IFNγ;
GPC-1, HGF and human monocyte chemotactic and activating factor (HuMCP1/MCAF);
GPC-1, HGF, and HuIL-18;
GPC-1, HGF, HuGM-CSF; GPC-1, HGF, and urokinase plasminogen activator (uPA);
GPC-1, HGF, and human melanoma growth stimulating activity alpha (HuGROa, also known as CXC motif ligand 1, CXCL1);
GPC-1, HGF and soluble vascular endothelial growth factor receptor (sVEGFR);
GPC-1, EGF and follistatin;
GPC-1, EGF and tumour necrosis factor alpha (TNFα); and
GPC-1, G-CSF and soluble tyrosine kinase with immunoglobulin like and EGF-like domains 2 (sTIE2).

Example 3. The method of embodiment 1 or embodiment 2, wherein said selecting the threshold value from the combined analyte levels comprises selecting a subset of the combined analyte levels.

Example 4. The method according to any one of embodiments 1 to 3, wherein said combining of the analyte levels in a manner that allows discrimination between the BPH and CaP samples comprises combining a weighted linear sum of these analyte levels in a manner that maximizes the discrimination between the BPH and CaP samples in accordance with the formula:

$$S = w_1A_1 + w_2A_2 \ldots w_nA_n$$

wherein S is the weighted sum,
w is the final analyte weighting,
and A is the level of a given analyte from n different analytes, or a transformation of the level of a given analyte either numerically or as a ratio of analyte values.

Example 5. The method of embodiment 4, wherein said transformation of the level of a given analyte numerically comprises use of an exponential function, a power function and/or a root function.

Example 6. The method according to embodiment 4 or embodiment 5, wherein obtaining the final analyte weightings w comprises generating initial analyte weightings (W) that are set to 1, or generated according to the formula:

$W = \max(M)/M$, where $W = \{w_1, ...w_n\}$ and $M = \{median(A_1), ...median(A_n)\}$;

wherein max (M) is a maximum median level of the analyte levels obtained, and M is a vector containing the median analyte level obtained for each said analyte.

Example 7. The method according to embodiment 6, wherein each said final weight is determined using a discriminating function for optimization in discerning between BPH and CaP in the series of biological samples.

Example 8. The method according to embodiment 7, wherein the discriminating function is any one or more of:

(i) the area under a curve generated by a ROC analysis;
(ii) a combination of True Positive Rates (TPR) and True Negative Rates (TNR);
(iii) the area under a curve generated by a ROC analysis within a restricted specificity range;
(iv) the area under a curve generated by a ROC analysis within a restricted sensitivity range.

Example 9. The method according to embodiment 7 or embodiment 8, wherein the discriminating function for optimization is any one or more of the Nelder-Mead (simplex method), a stochastic method, a gradient descent method, a stochastic gradient descent method, a genetic algorithm, a particle-swarm method, and/or a brute force method.

Example 10. The method according to any one of embodiments 1 to 9, wherein the suitable algorithm and/or transformation applied to the individual or combined analyte levels obtained from the subject's biological sample is in accordance with the formula:

$$S = w_1A_1 + w_2A_2 ... w_nA_n$$

wherein S is the weighted sum,
w is the final analyte weighting,
and A is the level of a given analyte from n different analytes, or a transformation of the level of a given analyte either numerically or as a ratio of analyte values.

Example 11. The method according to any one of embodiments 1 to 10, wherein said combining the analyte levels maximizes discrimination between the BPH and CaP samples.

Example 12. The method of any one of embodiments 1 to 11, comprising selecting the threshold value from the combined analyte levels in a manner that:

(i) reduces the misclassification rate between BPH and CaP; and/or
(ii) increases sensitivity in discriminating between BPH and CaP; and/or
(iii) increases specificity in discriminating between BPH and CaP.

Example 13. The method of embodiment 12, wherein said selecting the threshold value from the combined analyte levels in a manner that reduces the misclassification rate between BPH and CaP comprises selecting a suitable true positive and/or true negative rate.

Example 14. The method embodiment 12 or embodiment 13, wherein said selecting the threshold value from the combined analyte levels in a manner that reduces the misclassification rate between BPH and CaP minimizes the misclassification rate.

Example 15. The method according to any one of embodiments 12 to 14, wherein selecting the threshold value from the combined analyte levels in a manner that reduces the misclassification rate between BPH and CaP comprises minimizing the misclassification rate between BPH and CaP by identifying a point where the true positive rate intersects the true negative rate.

Example 16. The method according to embodiment 12, wherein selecting the threshold value from the combined analyte levels in a manner that increases sensitivity in discriminating between BPH and CaP comprises using a weighted sum Nelder-Mead optimization procedure to optimize for a partial area under a curve generated by a ROC analysis (pAUC), wherein the pAUC represents the area under the ROC within a restricted (1- sensitivity) range that is between value 0 and a specified value e (e.g. 0.5).

Example 17. The method embodiment 12 or embodiment 16 wherein said selecting the threshold value from the combined analyte levels in a manner that increases sensitivity in discriminating between BPH and CaP maximizes said sensitivity.

Example 18. The method embodiment 12 or embodiment 16 wherein said selecting the threshold value from the combined analyte levels in a manner that increases specificity in discriminating between BPH and CaP maximizes said specificity.

Example 19. The method according to embodiment 12 or embodiment 18, wherein selecting the threshold value from the combined analyte levels in a manner that increases specificity in discriminating between BPH and CaP comprises using a weighted sum Nelder-Mead optimization procedure to optimize for a partial area under a curve generated by a ROC analysis (pAUC), wherein the pAUC represents the area under the ROC within a restricted (1-specificity) range that is between values 0 and a specified value e (e.g. 0.5).

Example 20. The method according to any one of embodiments 12, 16, 17, 18 or 19, wherein the at least two analytes are selected from any one of the following analyte combinations:

GPC-1/EGF/follistatin; and
GPC-1/EGF/TNF$\alpha$.

Example 21. The method according to any one of embodiments 12, 16, 17, 18 or 19, wherein the at least two analytes are selected from any one of the following analyte combinations:

GPC-1/HGF;
GPC-1/HGF/P AI-1;
GPC-1/HGF/HuFGFb;
GPC-1/HGF/EGF;
GPC-1/HGF/HuPDFGBB;
GPC-1/EGF;
GPC-1/PAI-1;
GPC-1/G-CSF; and
GPC-1/PDGFAB.BB.

Example 22. The method according to any one of embodiments 1 to 19, wherein the at least two analytes are selected from any one of the following analyte combinations:

GPC-1/HGF;
GPC-1/PAI-1;
GPC-1/G-CSF;
GPC-1/EGF;
GPC-1/PDGF AB.BB;
GPC-1/HGF/FGFb;
GPC-1/HGF/PAI-1;
GPC-1/HGF/EGF;
GPC-1/HGF/Follistatin; and
GPC-1/HGF/G-CSF.

Example 23. The method according to any one of embodiments 1 to 22, wherein the subject has previously received a positive indication of prostate disease.

Embodiment 24. The method according to any one of embodiments 1 to 23, wherein the subject has previously received a positive indication of prostate disease by digital rectal exam (DRE) and/or by PSA testing.

Example 25. The method according to any one of embodiments 1 to 24, wherein said detecting at least two analytes in the subject's biological sample comprises:

(i) measuring one or more fluorescent signals indicative of each said analyte level;
(ii) obtaining a measurement of weight/volume of said analytes in the samples;
(iii) measuring an absorbance signal indicative of each said analyte level; or
(iv) using a technique selected from the group consisting of: mass spectrometry, a protein array technique, high performance liquid chromatography (HPLC), gel electrophoresis, radiolabeling, and any combination thereof.

Example 26. The method according to any one of embodiments 1 to 25, wherein said obtaining an analyte level in the biological samples of the series and the subject's biological sample comprises contacting each said sample with first and second antibody populations, wherein each said antibody population has binding specificity for one of said analytes, and the first and second antibody populations have different binding specificities.

Example 27. The method according to embodiment 26, wherein the first and/or second antibody populations are labelled.

Example 28. The method according to embodiment 27, wherein said label is selected from the group consisting of a radiolabel, a fluorescent label, a biotin-avidin amplification system, a chemiluminescence system, microspheres, and colloidal gold.

Example 29. The method according to any one of embodiments 26 to 28, wherein binding of each said antibody population to the analyte is detected by a technique selected from the group consisting of: immunofluorescence, radiolabeling, immunoblotting, Western blotting, enzyme-linked immunosorbent assay (ELISA), flow cytometry, immunoprecipitation, immunohistochemistry, biofilm test, affinity ring test, antibody array optical density test, and chemiluminescence.

Example 30. The method according to any one of embodiments 1 to 29, wherein the biological samples of the series and the subject's biological sample are each whole blood, serum, plasma, saliva, tear/s, urine, or tissue.

Example 31. The method according to any one of embodiments 1 to 30, wherein said subject, said population of subjects having BPH, and said population of subjects having CaP, are human.

Example 32. The method of any one of embodiments 1 to 31, wherein said detecting of the at least two analytes in the biological samples of the series and/or the subject's biological sample comprises detecting the analytes directly.

Example 33. The method of any one of embodiments 1 to 32, wherein said detecting of the at least two analytes in the biological samples of the series and/or the subject's biological sample comprises detecting a nucleic acid encoding the analytes.

Example 34. The method of any one of embodiments 1 to 33, wherein said detecting of the at least two analytes in the biological sample from the subject is performed *in vitro* or *ex vivo*.

**Definitions**

As used in this application, the singular form "a", "an" and "the" include plural references unless the context clearly dictates otherwise. For example, the phrase "an antibody" also includes multiple antibodies.

As used herein, the term "comprising" means "including". Variations of the word "comprising", such as "comprise" and "comprises," have correspondingly varied meanings. Thus, for example, a biomarker combination "comprising" analyte A and analyte B may consist exclusively of analyte A and analyte B or may include one or more additional components (e.g. analyte C).

As used herein, the term "prostate disease" refers to a disease of the prostate including, but not limited to, benign prostatic hyperplasia (BPH), prostatic intraepithelial neoplasia, prostatitis, prostate cancer, prostatic adenocarcinoma, prostatic leiomyosarcoma and prostatic rhabdomyosarcoma.

As used herein, the terms "biological sample" and "sample" encompass any body fluid or tissue taken from a subject including, but not limited to, a saliva sample, a tear sample, a blood sample, a serum sample, a plasma sample, a urine sample, or sub-fractions thereof.

As used herein, the terms "diagnosing" and "diagnosis" refer to methods by which a person of ordinary skill in the art can estimate and even determine whether or not a subject is suffering from a given disease or condition. A diagnosis may be made, for example, on the basis of one or more diagnostic indicators, such as for example, the detection of a biomarker or a combination of biomarkers as described herein, the levels of which are indicative of the presence, severity, or absence of the condition. As such, the terms "diagnosing" and "diagnosis" thus also include identifying a risk of developing prostate cancer.

As used herein, the terms "subject" and "patient" are used interchangeably unless otherwise indicated, and encompass any animal of economic, social or research importance including bovine, equine, ovine, primate, avian and rodent species. Hence, a "subject" may be a mammal such as, for example, a human or a non-human mammal. As used herein, the term "isolated" in reference to a biological molecule (e.g. an antibody) is a biological molecule that is free from at least some of the components with which it naturally occurs.

As used herein, the terms "antibody" and "antibodies" include IgG (including IgG1, IgG2, IgG3, and IgG4), IgA (including IgA1 and IgA2), IgD, IgE, IgM, and IgY, whole antibodies, including single-chain whole antibodies, and antigen-binding fragments thereof. Antigen-binding antibody fragments include, but are not limited to, Fv, Fab, Fab' and F(ab')2, Fd, single-chain Fvs (scFv), single-chain antibodies, disulfide-linked Fvs (sdFv) and fragments comprising either a VL or VH domain. The antibodies may be from any animal origin or appropriate production host. Antigen-binding antibody fragments, including single-chain antibodies, may comprise the variable region/s alone or in combination with the entire or partial of the following: hinge region, CH1, CH2, and CH3 domains. Also included are any combinations of variable region/s and hinge region, CH1, CH2, and CH3 domains. Antibodies may be monoclonal, polyclonal, chimeric, multispecific, humanized, or human monoclonal and polyclonal antibodies which specifically bind the biological molecule. The antibody may be a bi-specific antibody, avibody, diabody, tribody, tetrabody, nanobody, single domain antibody, VHH domain, human antibody, fully humanized antibody, partially humanized antibody, anticalin, adnectin, or affibody.

As used herein, the terms "binding specifically" and "specifically binding" in reference to an antibody, antibody variant, antibody derivative, antigen binding fragment, and the like refers to its capacity to bind to a given target molecule preferentially over other non-target molecules. For example, if the antibody, antibody variant, antibody derivative, or antigen binding fragment ("molecule A") is capable of "binding specifically" or "specifically binding" to a given target molecule ("molecule B"), molecule A has the capacity to discriminate between molecule B and any other number of potential alternative binding partners. Accordingly, when exposed to a plurality of different but equally accessible molecules as potential binding partners, molecule A will selectively bind to molecule B and other alternative potential binding partners will remain substantially unbound by molecule A. In general, molecule A will preferentially bind to molecule B at least 10-fold, preferably 50-fold, more preferably 100-fold, and most preferably greater than 100-fold more frequently than other potential binding partners. Molecule A may be capable of binding to molecules that are not molecule B at a weak, yet detectable level. This is commonly known as background binding and is readily discernible from molecule B-specific binding, for example, by use of an appropriate control.

As used herein, the term "kit" refers to any delivery system for delivering materials. Such delivery systems include systems that allow for the storage, transport, or delivery of reaction reagents (for example labels, reference samples, supporting material, etc. in the appropriate containers) and/or supporting materials (for example, buffers, written instructions for performing an assay etc.) from one location to another. For example, kits may include one or more enclosures, such as boxes, containing the relevant reaction reagents and/or supporting materials.

It will be understood that use of the term "between" herein when referring to a range of numerical values encompasses the numerical values at each endpoint of the range. For example, a polypeptide of between 10 residues and 20 residues in length is inclusive of a polypeptide of 10 residues in length and a polypeptide of 20 residues in length.

Any description of prior art documents herein, or statements herein derived from or based on those documents, is not an admission that the documents or derived statements are part of the common general knowledge of the relevant art.

## Detailed Description

The development of reliable, convenient, and accurate tests for the diagnosis of prostate disease remains an important objective, particularly during early stages when therapeutic intervention has the highest chance of success. However, initial screening procedures such as DRE and PSA assays often provide uncertain/inconclusive diagnostic outcomes and are unable to effectively discern between cancerous and non-cancerous prostate disease.

The present invention provides biomarker combinations indicative of prostate disease. In particular, the biomarker combinations may be capable of discerning between non-cancerous prostate disease (e.g. BPH) and prostate cancer in a given subject. The subject may have previously been determined to have a form of prostate disease, or be suspected of having a form of prostate disease, on the basis of one or more diagnostic tests (e.g. DRE, PSA testing). The biomarker combinations may thus be used in various methods and assay formats to diagnose the presence or absence of prostate cancer or BPH in subjects and/or to differentiate BPH patients from patients with prostate cancer.

### *Prostate Diseases*

The present disclosure provides methods for the diagnosis of prostate disease. The methods involve detection of one or more biomarker combination/s as described herein.

A prostate disease diagnosed in accordance with the methods may include, but is not limited to, benign prostatic hyperplasia (BPH), prostatic intraepithelial neoplasia, prostatitis, prostate cancer, prostatic adenocarcinoma, prostatic leiomyosarcoma and prostatic rhabdomyosarcoma.

In certain examples, the methods provide a capacity to distinguish between "normal" patients who are entirely or significantly free of cancerous prostate disease, and subjects with prostate cancer. The normal patients may have a non-cancerous prostate disease such as, for example, BPH, prostatic intraepithelial neoplasia (PIN), or prostatitis.

Persons of ordinary skill in the art are well aware of standard clinical tests and parameters used to classify different prostate diseases (see, for example, *"2015 Annual Report on Prostate Diseases"*, Harvard Health Publications (Harvard Medical School), 2015;).

The various prostate diseases contemplated in the present disclosure have the features and classification criteria consistent with those generally accepted in the field.

For example, as contemplated herein benign prostatic hyperplasia (BPH) can be understood to refer to a clinical enlargement of the prostate which in some cases culminates in lower urinary tract symptoms including, but not limited to, frequent urination with an increased residual urine volume, urinary retention, and an irritative or obstructed urine voiding pattern.

Prostatitis can be understood to refer to a disorder associated with inflammation of the prostate, including chronic non-bacterial prostatitis and acute or chronic bacterial prostatitis, and which is generally associated with symptoms of urinary urgency and/or urinary frequency.

Prostatic intraepithelial neoplasia (PIN) can be understood to encompass various forms and/or degrees of PIN including, but not limited to, high grade prostatic intraepithelial neoplasia and low grade prostatic intraepithelial neoplasia. PIN can be considered a precancerous condition in which some prostate cells have begun to look and/or behave abnormally. The abnormal cells can be located, for example, in epithelial cells lining the acini and ducts whereas the lining itself can remain intact.

In subjects with prostate cancer, cells of the prostate divide indefinitely causing abnormal growths (e.g. tumours). The abnormal growths may be painless and asymptomatic. Alternatively, the abnormal growths may cause physical discomfort and/or other localized symptoms including fluid blockages or bleeding. Additionally or alternatively, the abnormal growths may culminate in systemic symptoms including those arising from disruption of normal body functions. In some embodiments the symptoms of prostate cancer may include change in bowel habits or bladder function. The prostate cancer cells may be benign or metastatic.

As known to those of ordinary skill in the art, prostate cancer can be categorized into stages according to the progression of the disease. By way of non-limiting example, prostate cancer progression may be categorized into stages using the AJCC TNM staging system, the Whitmore-Jewett system and/or the D'Amico risk categories.

Ordinarily skilled persons in the field are familiar with such classification systems, their features and their use.

A suitable system of grading prostate cancer also known to those of ordinary skill in the field as the "Gleason Grading System". This system assigns a grade to each of the two largest areas of cancer in tissue samples obtained from a subject with prostate cancer. The grades range from 1-5, 1 being the least aggressive form and 5 the most aggressive form. Metastases are common with grade 4 or grade 5, but seldom occur, for example, in grade 3 tumors. The two grades are then added together to produce a Gleason score. A score of 2-4 is considered low grade; 5-7 intermediate grade; and 8-10 high grade. A tumor with a low Gleason score may typically grow at a slow enough rate to not pose a significant threat to the patient during their lifetime.

As known to those skilled in the art, prostate cancers may have areas with different grades in which case individual grades may be assigned to the two areas that make up most of the prostate cancer. These two grades are added to yield the Gleason score/sum, and in general the first number assigned is the grade which is most common in the tumour. For example, if the Gleason score/sum is written as '3+4', it means most of the tumour is grade 3 and less is grade 4, for a Gleason score/sum of 7.

By way of non-limiting example, a Gleason score/sum of 3+4 and above may be indicative of prostate cancer according to the methods of the present invention.

### *Biomarkers for Prostate Disease*

In accordance with the methods of the present disclosure, prostate diseases can be diagnosed by way of detecting one or more biomarkers identified herein.

A biomarker as contemplated herein may be an analyte. An analyte as contemplated herein is to be given its ordinary and customary meaning to a person of ordinary skill in the art and refers without limitation to a substance or chemical constituent in a biological sample (for example, blood, cerebral spinal fluid, urine, tear/s, lymph fluid, saliva, interstitial fluid, sweat, etc.) that can be detected and quantified. Non-limiting examples include cytokines and chemokines, as well as cell-surface receptors and soluble forms thereof.

A combination of biomarkers detected in accordance may comprise or consist of two, three, four, five, or more than five individual biomarkers. Accordingly, a combination of biomarkers may comprise GPC-1 and at least one additional biomarker, GPC-1 and at least two additional biomarkers, or GPC-1 and at least three additional biomarkers. One or more of the additional biomarkers may be analytes.

In some examples, a combination of at least two biomarkers in a biological sample of a subject may be measured and used as a basis to derive a positive or negative diagnosis for prostate disease. The combination of at least two biomarkers may comprise GPC-1. The prostate disease may be prostate cancer or BPH. By way of non-limiting example, the combination of at least two biomarkers may be selected from those set out in **Table 1** below.

**Table 1: combinations of two biomarkers.**

| Biomarker #1 | Biomarker #2 |
|---|---|
| GPC-1 | HGF |
| GPC-1 | EGF |
| GPC-1 | PAI-1 |

(continued)

| Biomarker #1 | Biomarker #2 |
| --- | --- |
| GPC-1 | G-CSF |
| GPC-1 | HuIL-18 |
| GPC-1 | PDGFAB.BB |
| GPC-1 | PDGFBB |
| GPC-1 | sCD40L |
| GPC-1 | HuGM-CSF |
| GPC-1 | IFNγ |
| GPC-1 | Follistatin |

In some examples, a combination of at least three biomarkers in a biological sample of a subject may be measured and used as a basis to derive a positive or negative diagnosis for prostate disease. The combination of at least three biomarkers may comprise GPC-1. The prostate disease may be prostate cancer or BPH. By way of non-limiting example, the combination of at least three biomarkers may be selected from those set out in **Table 2** below.

**Table 2: combinations of three biomarkers.**

| Biomarker #1 | Biomarker #2 | Biomarker #3 |
| --- | --- | --- |
| GPC-1 | HGF | PAI-1 |
| GPC-1 | HGF | HuFGFb |
| GPC-1 | HGF | EGF |
| GPC-1 | HGF | HuPDGFBB |
| GPC-1 | HGF | Follistatin |
| GPC-1 | HGF | G-CSF |
| GPC-1 | HGF | sCD40L |
| GPC-1 | HGF | HuIL-8 |
| GPC-1 | HGF | HuCTACK |
| GPC-1 | HGF | PDGFAB.BB |
| GPC-1 | HGF | IFNγ |
| GPC-1 | HGF | HuMCP1 MCAF |
| GPC-1 | HGF | HuIL-18 |
| GPC-1 | HGF | HuGM-CSF |
| GPC-1 | HGF | uPA |
| GPC-1 | HGF | HuGROa |
| GPC-1 | HGF | sVEGFR |
| GPC-1 | EGF | Follistatin |
| GPC-1 | EGF | TNFα |
| GPC-1 | G-CSF | sTIE2 |

*Detection and Quantification of Biomarkers*

A biomarker or combination of biomarkers according to the present invention may be detected in a biological sample using any suitable method known to those of ordinary skill in the art.

In some embodiments, the biomarker or combination of biomarkers is quantified to derive a specific level of the biomarker or combination of biomarkers in the sample. Level/s of the biomarker/s can be analyzed according to the methods provided herein to provide a diagnosis.

Detecting the biomarker/s in a given biological sample may provide an output capable of measurement, thus providing a means of quantifying the levels of the biomarker/s present. Measurement of the output signal may be used to generate a figure indicative of the net weight of the biomarker per volume of the biological sample (e.g. pg/mL; µg/mL; ng/mL etc.).

By way of non-limiting example only, detection of the biomarker/s may culminate in one or more fluorescent signals indicative of the level of the biomarker/s in the sample. These fluorescent signals may be used directly to make a diagnostic determination according to the methods of the present invention, or alternatively be converted into a different output for that same purpose (e.g. a weight per volume as set out in the paragraph directly above).

Biomarkers according to the present invention can be detected and quantified using suitable methods known in the art including, for example, proteomic techniques and techniques which utilise nucleic acids encoding the biomarkers.

Non-limiting examples of suitable proteomic techniques include mass spectrometry, protein array techniques (e.g. protein chips), gel electrophoresis, and other methods relying on antibodies having specificity for the biomarker/s including immunofluorescence, radiolabeling, immunohistochemistry, immunoprecipitation, Western blot analysis, enzyme-linked immunosorbent assays (ELISA), fluorescent cell sorting (FACS), immunoblotting, chemiluminescence, and/or other known techniques used to detect protein with antibodies.

Non-limiting examples of suitable techniques relying on nucleic acid detection include those that detect DNA, RNA (e.g. mRNA), cDNA and the like, such as PCR-based techniques (e.g. quantitative real-time PCR; SYBR-green dye staining), UV spectrometry, hybridisation assays (e.g. slot blot hybridisation), and microarrays.

Antibodies having binding specificity for a biomarker according to the present invention, including monoclonal and polyclonal antibodies, are readily available and can be purchased from a variety of commercial sources (e.g. Sigma-Aldrich, Santa Cruz Biotechnology, Abcam, Abnova etc.). Additionally or alternatively, antibodies having binding specificity for a biomarker according to the present invention can be produced using standard methodologies in the art. Techniques for the production of hybridoma cells capable of producing monoclonal antibodies are well known in the field. Non-limiting examples include the hybridoma method (see Kohler and Milstein, (1975) Nature, 256:495-497; Coligan et al. section 2.5.1-2.6.7 in Methods In Molecular Biology (Humana Press 1992); and Harlow and Lane Antibodies: A Laboratory Manual, page 726 (Cold Spring Harbor Pub. 1988)), the EBV-hybridoma method for producing human monoclonal antibodies (see Cole, et al. 1985, in Monoclonal Antibodies and Cancer Therapy, Alan R. Liss, Inc., pp. 77-96), the human B-cell hybridoma technique (see Kozbor et al. 1983, Immunology Today 4:72), and the trioma technique.

In some embodiments, detection/quantification of the biomarker/s in a biological sample (e.g. a body fluid or tissue sample) is achieved using an Enzyme-linked immunosorbent assay (ELISA). The ELISA may, for example, be based on colourimetry, chemiluminescence, and/or fluorometry. An ELISA suitable for use in the methods of the present invention may employ any suitable capture reagent and detectable reagent including antibodies and derivatives thereof, protein ligands and the like.

By way of non-limiting example, in a direct ELISA the biomarker of interest can be immobilised by direct adsorption onto an assay plate or by using a capture antibody attached to the plate surface. Detection of the antigen can then be performed using an enzyme-conjugated primary antibody (direct detection) or a matched set of unlabeled primary and conjugated secondary antibodies (indirect detection). The indirect detection method may utilise a labeled secondary antibody for detection having binding specificity for the primary antibody. The capture (if used) and/or primary antibodies may derive from different host species.

In some embodiments, the ELISA is a competitive ELISA, a sandwich ELISA, an in-cell ELISA, or an ELISPOT (enzyme-linked immunospot assay).

Methods for preparing and performing ELISAs are well known to those of ordinary skill in the art. Procedural considerations such as the selection and coating of ELISA plates, the use of appropriate antibodies or probes, the use of blocking buffers and wash buffers, the specifics of the detection step (e.g. radioactive or fluorescent tags, enzyme substrates and the like), are well established and routine in the field (see, for example, "The Immunoassay Handbook. Theory and applications of ligand binding, ELISA and related techniques", Wild, D. (Ed), 4th edition, 2013, Elsevier).

In other embodiments, detection/quantification of the biomarker/s in a biological sample (e.g. a body fluid or tissue sample) is achieved using Western blotting. Western blotting is well known to those of ordinary skill in the art (see for example, Harlow and Lane. Using antibodies. A Laboratory Manual. Cold Spring Harbor, New York: Cold Spring Harbor Laboratory Press, 1999; Bold and Mahoney, Analytical Biochemistry 257, 185-192, 1997). Briefly, antibodies having binding affinity to a given biomarker can be used to quantify the biomarker in a mixture of proteins that have been separated based on size by gel electrophoresis. A membrane made of, for example, nitrocellulose or polyvinylidene fluoride (PVDF) can be placed next to a gel comprising a protein mixture from a biological sample and an electrical current applied to induce the proteins to migrate from the gel to the membrane. The membrane can then be contacted with antibodies having specificity for a biomarker of interest, and visualised using secondary antibodies and/or detection reagents.

In other embodiments, detection/quantification of multiple biomarkers in a biological sample (e.g. a body fluid or tissue sample) is achieved using a multiplex protein assay (e.g. a planar assay or a bead-based assay). There are numerous multiplex protein assay formats commercially available (e.g. Bio-rad, Luminex, EMD Millipore), and non-limiting examples of suitable multiplex protein assays are described in the Examples section of the present specification.

In other embodiments, detection/quantification of biomarker/s in a biological sample (e.g. a body fluid or tissue sample) is achieved by flow cytometry, which is a technique for counting, examining and sorting target entities (e.g. cells and proteins) suspended in a stream of fluid. It allows simultaneous multiparametric analysis of the physical and/or chemical characteristics of entities flowing through an optical/electronic detection apparatus (e.g. target biomarker/s quantification).

In other embodiments, detection/quantification of biomarker/s in a biological sample (e.g. a body fluid or tissue sample) is achieved by immunohistochemistry or immunocytochemistry, which are processes of localising proteins in a tissue section or cell, by use of antibodies or protein binding agent having binding specificity for antigens in tissue or cells. Visualisation may be enabled by tagging the antibody/agent with labels that produce colour (e.g. horseradish peroxidase and alkaline phosphatase) or fluorescence (e.g. fluorescein isothiocyanate (FITC) or phycoerythrin (PE)).

Persons of ordinary skill in the art will recognise that the particular method used to detect biomarker/s according to the present invention or nucleic acids encoding them is a matter of routine choice that does not require inventive input.

### *Analysis of Biomarkers and Diagnosis*

According to methods of the present invention, detection and quantification of a given biomarker or a given combination of biomarkers can be used to diagnose prostate diseases.

Generally, the methods involve analysing the targeted biomarker/s in a given biological sample or a series of biological samples to derive a quantitative measure of the biomarker/s in the sample. Suitable biomarker/s include, but are not limited to, those biomarkers and biomarker combinations referred to in **Tables 1** and **2**. By way of non-limiting example only, the quantitative measure may be in the form of a fluorescent signal or an absorbance signal as generated by an assay designed to detect and quantify the biomarker/s. Additionally or alternatively, the quantitative measure may be provided in the form of weight/volume measurements of the biomarker/s in the sample/s.

In some embodiments, the methods of the present invention may comprise a comparison of levels of the biomarker/s in patient populations known to suffer from a prostate disease as described herein. For example, levels of biomarker/s can be ascertained from a series of biological samples obtained from patients having a first prostate disease and from a series of biological samples obtained from patients having a second prostate disease. The first prostate disease may be a non-cancerous prostate disease such BPH. The second prostate disease may be prostate cancer including forms thereof such as any one or more of prostatic adenocarcinoma, prostatic leiomyosarcoma and prostatic rhabdomyosarcoma. The prostate cancer may be characterised by a minimum Gleason grade or score/sum (e.g. at least 6 (e.g. 3+3, 4+2 or 2+4); at least 7 (e.g. 3+4 or 4+3, 5+2), or at least 8 (e.g. 4+4, 5+3; or 3+5).

The level of biomarker/s observed in samples from each individual population may be collectively analyzed to determine a threshold value that can be used as a basis to provide a diagnosis of cancerous or non-cancerous prostate disease. For example, a biological sample from a patient confirmed or suspected to be suffering from a prostate disease can be analyzed and the levels of target biomarker/s according to the present invention determined. Comparison of levels of the biomarker/s in the patient's sample to the threshold value/s generated from the patient populations can serve as a basis to diagnose cancerous or non-cancerous prostate disease.

Accordingly, in some embodiments the methods of the present invention comprise diagnosing whether a given patient suffers from a non-cancerous prostate disease or a cancerous prostate disease. The patient may have been previously confirmed to have or may be suspected of having a prostate disease, for example, as a result of a DRE and/or PSA test. In such situations, it is advantageous for the patient to determine whether the prostate disease is cancerous or not, as a diagnosis of non-cancerous prostate disease in accordance with the methods described herein avoids the need for a prostate biopsy.

Without any particular limitation, a diagnostic method may involve discerning whether a subject has a specific form of prostate disease. The method may comprise obtaining a first series of biological samples from a first group of patients pre-determined to be suffering from prostate disease A, and a second series of biological samples from a second group of patients pre-determined not to be suffering from prostate disease A. The second group of patients may suffer from a different prostate disease (e.g. prostate disease B), or alternatively not suffer from any prostate disease (prostate disease-). A threshold value for discerning between the first and second patient groups may be generated by detecting one, two, three, four, five or more than five biomarkers in the first and second series of biological samples to thereby obtain a biomarker level for each biomarker in each biological sample of each series. The analyte levels may be combined in a manner that allows discrimination between samples from the first and second group of patients. A threshold value may be selected from the combined analyte levels in a suitable manner such as any one or more of a method that:

reduces the misclassification rate between the first and second group of patients; increases or maximizes the sensitivity in discriminating between the first and second group of patients; and/or increases or maximizes the specificity in discriminating between the first and second group of patients. The threshold value can be used as a basis to discriminate between the presence and absence of prostate disease A in a given candidate sample. Hence, a biological sample from a subject who's status in relation to prostate disease A is undetermined may be obtained and the same biomarker/s that served as the basis for generating the threshold value measured in the same manner as for the first and second patient groups to obtain a patient biomarker value. The patient biomarker value derived from the quantified biomarker level/s can then be compared to the threshold value for a determination of prostate disease A to be made. A suitable algorithm and/or transformation of individual or combined biomarker levels obtained from the subject's biological sample may be used to generate the patient biomarker value for comparison to the threshold value. In some embodiments, one or more parameters used in deriving the threshold value and/or the patient biomarker value are weighted.

In some embodiments, the patient receives a negative diagnosis for prostate disease A if the patient biomarker value is less than the threshold value. In some embodiments, the patient receives a negative diagnosis for prostate disease A if the patient biomarker value is more than the threshold value. In some embodiments, the patient receives a positive diagnosis for prostate disease A if the patient biomarker value is less than the threshold value. In some embodiments, the patient receives a positive diagnosis for prostate disease A if the patient biomarker value is more than the threshold value. In some embodiments, prostate disease A is a non-cancerous prostate disease (e.g. BPH). In some embodiments, prostate disease A is a prostate cancer, prostatic adenocarcinoma, prostatic leiomyosarcoma and/or prostatic rhabdomyosarcoma.

Suitable and non-limiting methods for conducting these analyses are described in the Examples of the present application.

One non-limiting example of such a method is Receiver Operating Characteristic (ROC) curve analysis. Generally, the ROC analysis may involve comparing a classification for each patient tested to a 'true' classification based on an appropriate reference standard. Classification of multiple patients in this manner may allow derivation of measures of sensitivity and specificity. Sensitivity will generally be the proportion of correctly classified patients among all of those that are truly positive, and specificity the proportion of correctly classified cases among all of those that are truly negative. In general, a trade-off may exist between sensitivity and specificity depending on the threshold value selected for determining a positive classification. A low threshold may generally have a high sensitivity but relatively low specificity. In contrast, a high threshold may generally have a low sensitivity but a relatively high specificity. A ROC curve may be generated by inverting a plot of sensitivity versus specificity horizontally. The resulting inverted horizontal axis is the false positive fraction, which is equal to the specificity subtracted from 1. The area under the ROC curve (AUC) may be interpreted as the average sensitivity over the entire range of possible specificities, or the average specificity over the entire range of possible sensitivities. The AUC represents an overall accuracy measure and also represents an accuracy measure covering all possible interpretation thresholds.

While methods employing an analysis of the entire ROC curve are encompassed, it is also intended that the methods may be extended to statistical analysis of a partial area (partial AUC analysis). The choice of the appropriate range along the horizontal or vertical axis in a partial AUC analysis may depend at least in part on the clinical purpose. In a clinical setting in which it is important to detect the presence of a given prostate disease with high accuracy, a range of relatively high false positive fractions corresponding to high sensitivity may be used. Alternatively, in a clinical setting in which it is important to exclude a given prostate disease, a range of relatively low false positive fractions equivalent to high specificities may be used.


### Subjects, Samples and Controls

Samples analyzed according to the methods of the present invention are derived from one or a series of subjects. A subject or patient referred to herein encompasses any animal of economic, social or research importance including bovine, equine, ovine, primate, avian and rodent species. A subject or patient may be a mammal such as, for example, a human or a non-human mammal. Subjects and patients as described herein may or may not suffer from prostate disease, may or may not suffer from a cancerous prostate disease, or may or may not suffer from a non-cancerous prostate disease.

A sample used in accordance the methods of the present invention may be in a form suitable to allow analysis by the skilled artisan. Suitable samples include various body fluids such as blood, plasma, serum, semen, urine, tear/s, cerebral spinal fluid, lymph fluid, saliva, interstitial fluid, sweat, etc. The urine may be obtained following massaging of the prostate gland.

The sample may be a tissue sample, such as a biopsy of the tissue, or a superficial sample scraped from the tissue. The tissue may be from the prostate gland. In another embodiment the sample may be prepared by forming a suspension of cells made from the tissue.

The methods of the present invention may, in some embodiments, involve the use of control samples.

A control sample is any corresponding sample (tissue sample, blood, plasma, serum, semen, tear/s, or urine) that is taken from an individual without prostate disease. In certain embodiments, the control sample may comprise or consist of nucleic acid material encoding a biomarker according to the present invention.

In some embodiments, the control sample can include a standard sample. The standard sample can provide reference amounts of biomarker at levels considered to be control levels. For example, a standard sample can be prepared to mimic the amounts or levels of a biomarker described herein in one or more samples (e.g. an average of amounts or levels from multiple samples) from one or more subjects, who may or may not have a prostate disease.

In some embodiments control data may be utilized. Control data, when used as a reference, can comprise compilations of data, such as may be contained in a table, chart, graph (e.g. database or standard curve) that provides amounts or levels of biomarker considered to be control levels. Such data can be compiled, for example, by obtaining amounts or levels of the biomarker in one or more samples (e.g. an average of amounts or levels from multiple samples) from one or more subjects, who may or may not have a prostate disease.

### *Kits*

Also contemplated herein are kits for performing the methods of the present invention.

The kits may comprise reagents suitable for detecting one or more biomarker/s described herein, including, but not limited to, those biomarker and biomarker combinations referred to in **Tables 1** and **2**.

By way of non-limiting example, the kits may comprise one or a series of antibodies capable of binding specifically to one or a series of biomarkers described herein.

Additionally or alternatively, the kits may comprise reagents and/or components for preparing and/or conducting assays capable of quantifying one or more biomarker/s described herein (e.g. reagents for performing an ELISA, flow cytometry, Western blot, immunohistochemistry, gel electrophoresis (as suitable for protein and/or nucleic acid separation) and/or quantitative PCR).

Additionally or alternatively, the kits may comprise equipment for obtaining and/or processing a biological sample as described herein, from a patient.

### EXAMPLES

The present invention will now be described with reference to specific Examples, which should not be construed as in any way limiting.

### Example 1: Analyte expression in benign prostatic hyperplasia [BPH] and prostate cancer [CaP] patients.

The expression of 82 analytes was investigated in plasma samples from normal patients (Arm 1), patients with Benign Prostatic Hyperplasia (BPH, ARM2) and patients with prostate cancer (CaP, Arm 3) using the Biorad Bioplex multi-analyte array system. The levels of soluble GPC-1 were determined using a sandwich ELISA consisting of a goat polyclonal anti-GPC-1 capture antibody and the MIL-38 anti-GPC-1 monoclonal detector antibody.

### *1. Materials and Methods*

*1.1 Patients*

Plasma samples from 298 patients were collected for multianalyte analysis. Samples from each of three groups were analyzed: normal (98 patients), benign prostatic hyperplasia [BPH, 100 patients] and prostate cancer [CaP, 100 patients].

Patient categories were defined as follows:

*Arm 1 - Normal*

*- INCLUSION*

Males 50 years and older, including those on alpha-blockers, with normal DRE and normal PSA diagnosed at for least 1 week and at most 1 year, without any manipulation of the lower urinary tract (such as endoscopy) except DRE in the previous 3 month and with PSA defined as:

- PSA < 2 ng/mL between ages 50 and 60
- PSA < 3 ng/mL above age 60

Typically such patients would consult for vasectomy, stent or stone procedures or erectile dysfunction.

*- EXCLUSION*

Any ARM 1 patient having undergone lower urinary tract manipulation other than DRE in previous 3 months was excluded Any ARM 1 patient without DRE result or with DRE not within approved timeframe was excluded.
Any ARM 1 patient without PSA result or with PSA not within approved timeframe was excluded.
Any ARM 1 patient with asymptomatic BPH was excluded from ARM 1 but could be considered for inclusion in ARM 2 (BPH).

*Arm 2 - Benign prostatic hyperplasia (BPH)*

*- INCLUSION*

Males 50 years and older, including those on alpha-blockers, with pathology-confirmed benign prostatic hyperplasia, within at least 4 weeks and at most 1 year. In addition, males 50 years and older with clinical BPH including Lower Urinary Tract Symptoms (LUTS) were included if PSA was within the age adjusted normal range and the PSA test was performed at least within 1 week and at most 12 months. Normal PSA was defined as PSA < 2 ng/mL between ages 50 and 60 and PSA < 3 ng/mL above age 60.

*- EXCLUSION*

Any patient in ARM 2 with PIN or atypia was excluded.

*Arm 3 - Prostate Cancer (CaP)*

*- INCLUSION*

Males 50 years and older, including those on alpha-blockers, at least 1 week post biopsy confirmed prostate cancer patients and Gleason score of at least 7 as Gleason 3+4 or Gleason 4+3.

*- EXCLUSION*

Any patient in ARM 3 (prostate cancer or CaP) having undergone partial or radical prostatectomy or other therapeutic procedure such as HIFU or brachytherapy was excluded.
Any patient in ARM 3 under 5 alpha reductase inhibitors (5ARI) was excluded unless there was a minimum of 2 days wash out since the last dose of finasteride and a minimum of 30 weeks wash out since the last dose of durasteride.
Any patient in ARM 3 under Saw Palmetto was excluded unless there was a minimum wash out of 1 month since last dose.
Any patient in ARM 3 under Androgen Deprivation Therapy (ADT) or any experimental agent was excluded.
Exclusion criteria for all arms were as follows:

- Any patient not meeting the inclusion criteria was excluded.
- Any patient with medical history of cancer other than prostate cancer except basal or squamous skin cancer was excluded.

*1.2 Sample collection*

Whole blood samples taken from patients were subjected to centrifugation to separate plasma components which were stored at -20°C. Samples were shipped from the collection sites then thawed, aliquoted and stored at -80°C.

*1.3 Multi-analyte arrays*

Patient plasma samples were defrosted on ice then transferred to a 1.5mL centrifuge tubes. The samples were spun at 20,000g for 10 mins at 4°C. The middle fraction was transferred, avoiding any pellet or lipid layer, to a 0.22μm centrifugal filtration device and spun at 11,000g at 4°C until complete. The samples were then aliquoted onto microtitre plates so they could be processed for the Bio-plex readings. The plates were stored at -80°C until they could be processed and run at the Australian Proteome Analysis Facility as per Bio-plex kit instructions. The samples were analyzed using a Bioplex 200 analyser according to manufacturer's instructions and as described in Breen *et al.* (see Breen et al. 2015, Cytokine 71, 188-198). The Bio-plex 200 system (Bio-Rad) is a flexible analyser based on the principles of flow cytometry. The system enables one to multiplex (simultaneously measure) up to 100 analytes in a single microplate well, using small sample volumes. Bio-Plex assays are designed using microscopic beads, each with a different colour code (spectral address) to permit discrimination of individual assays. Beads are dyed with different ratios of two fluorophores and are thus classified into 100 unique bead regions (xMAP technology).

Four kits were obtained from Biorad for this analysis:

Bio-Plex Pro™ Human Cytokine 21-plex Assay, Cat# MF0-005KMII
Bio-Plex Pro™ Human Cytokine 27-plex Assay, Cat# M50-0KCAF0Y
Bio-Plex Pro™ Human Cancer Biomarker Panel 1, 16-plex, Cat# 171-AC500M
Bio-Plex Pro™ Human Cancer Biomarker Panel 2, 18-plex, Cat# 171-AC600M

The cytokines and growth factors contained in each kit were as follows:

*27-plex:* IL-1beta, IL-1ra, IL-2, IL-4, IL-5, IL-6, IL-7, IL-8, IL-9, 1L-10, IL-12 (p70), IL-13, IL-15, IL-17, Basic FGF, Eotaxin, G-CSF, GM-CSF, IFN-gamma, IP-10, MCP-1 (MCAF), MIP-1alpha, MIP-1beta, PDGF-BB, RANTES, TNF-alpha and VEGF;
*21-plex:* IL-1alpha, IL-2Ralpha, IL-3, IL-12 (p70), IL-16, IL-18, CTACK, GRO-alpha, HGF, IFN-alpha2, LIF, MCP-3, M-CSF, MIF, MIG, beta-NGF. SCF, SCGF-beta, SDF-1alpha, TNF-beta and TRAIL;
*16-plex (Cancer panel 1):* sEGFR, FGF-basic, Follistatin, G-CSF, HGF, sHER-2/neu, IL-6R$\alpha$, Leptin, Osteopontin, PECAM-1, PDGF-AB/BB, Prolactin, SCF, sTIE-2, sVEGFR-1, sVEGFR-2;
*18-plex (Cancer panel 2):* Angiopoietin-2, sCD40L, EGF, Endoglin, sFASL, HB-EGF, IGFBP-1, L-6, IL-8, IL-18, PAI-1, PLGF, TGF-$\alpha$, TNF-$\alpha$, uPA, VEGF-A, VEGF-C, VEGF-D.

*1.4 MiCheck™ GPC-1 ELISA*

The MiCheck™ ELISA is a standard sandwich ELISA format in a 96 well plate. It employs a polyclonal anti-GPC-1 capture antibody (LSBio, Cat# LS-C313545) and the MIL-38 monoclonal antibody for detection. The assay is run in 2 hours and is compatible with all common automated ELISA systems.

Successful internal validation of the prototype MiCheck™ assay using serum and plasma has been completed. The results were as follows:

- **Linearity:** Assay linearity was demonstrated over a working concentration range of 10 pg/mL to 1.5 ng/mL.
- **Sensitivity:** Calculated limit of detection (LOD) and limit of quantitation (LOQ) of recombinant GPC-1 was approximately 3.4 pg/mL and 7.2 pg/mL respectively.
- **Human serum and plasma sample detection**: Assessed over a dilution range from 2 to 128 fold. There was good linearity of detection observed between 4-fold and 128-fold diluted serum/plasma - with an average dilution 10 to 20 fold. The average of interpolated values of GPC-1 were 5.4 ng/mL with 3.8% CV's across the various dilutions.
- **Precision:** The mean CVs were calculated from 3 concentrations (1.0/0.5/0.25 ng/mL) of GPC-1 analyzed within the working range of assay.

**Table 3: intra- and inter- assay performance of the MiCheck™ ELISA for serum and plasma.**

|  | Intra-Assay | Inter-Assay |
|---|---|---|
| n= | 3 | 3 |
| cv (%) | 4.3 | 2.3 |

*1.5 Data analysis*

For Bioplex analysis, samples were aliquoted according to a sequential internal numbering system in a downward direction in each column of a 96 well microtitre plate. Fluorescence values were determined and used for analysis.

ROC analysis (AUC)

ROC analysis was determined using the R statistical package (Robin et al. 2011, BMC Bioinformatics, 12, p77).

*Weighting model*

An algorithm was developed to find the best combination of analyte expression that differentiates benign and prostate cancer specimens. This model uses a weighted sum (S) of the expressions from n different analytes (A) as follows:

$$S = w_1 A_1 + w_2 A_2 \ldots w_n A_n$$

The weights $w_1$ to $w_n$ were determined using a general purpose optimization procedure, the Nelder-Mead algorithm in the R analysis program.

The procedure optimized for the maximum AUC of ROC analysis on the values of S to assign either benign or PC status.

The initial value for each weight was determined by taking the ratio between median expression values of each analyte to the maximum median from those analytes:

$$W = \max (M)/M$$

Where W = {$w_1$, ...$w_n$} and M = {median($A_1$), ...median($A_n$)}.

This model was utilized to optimize S for both two and three analyte combinations (see Pepe et al. 2003, Biometrics, 59:133).

*Partial AUC optimization*

An alternative approach was utilized whereby a weighted sum Nelder-Mead optimization procedure was applied to optimize for a partial AUC. pAUC represents the area under the ROC within a restricted (1-specificity) range, and generally this range lies between values 0 and e, where e must be specified. Here e was set to 0.5. A ROC that rises earlier than another ROC that rises later, when both AUCs are equal, will have higher specificity. Optimizing for pAUC rather than the full AUC optimizes for tests with higher specificity.

This approach was used to generate a weighted sum for N analytes using the R analysis program as described for the for the ROC AUC analysis.

### 2. Results

2.1 Analysis

*GPC-1 analysis*

Soluble GPC-1 levels were determined in normal, BPH and CaP patients using the MiCheck™ ELISA assay (**Table 4**). GPC-1 levels were lower in CaP patient samples than in either normal or BPH samples.

Table 4: GPC-1 ELISA results for Arm 1 (normal), Arm 2 (BPH) and Arm 3 (CaP).

| Measure | Study Arm | N = | Mean (ng/ml) | Std Dev | Range |
|---|---|---|---|---|---|
| **Plasma** | 1 | 98 | 8.0 | 2.0 | 4.3-15.8 |
| | 2 | 100 | 8.2 | 2.4 | 3.5-17.1 |
| | 3 | 100 | 7.4 | 1.9 | 4.2-14.6 |
| **Serum** | 1 | 99 | 8.4 | 2.0 | 3.7-17.3 |
| | 2 | 100 | 8.7 | 2.7 | 4.9-17.6 |
| | 3 | 99 | 7.8 | 2.1 | 4.4-17.0 |

GPC-1 levels were log transformed to achieve a normal data distribution. Sensitivity and specificity of the MiCheck™ ELISA to discriminate between BPH and CaP groups were determined at different cutpoints (**Table 5**).

The log GPC-1 levels gave a maximum AUC of 0.585 at a cutpoint of 1.95 and sensitivity and specificity of 0.51 (51%) and 0.66 (61%) respectively.

A second independent analysis of the data yielded an AUC of 0.61 and sensitivity/specificity of 0.57/0.57. Sensitivities at different fixed specificities were 20% at 90% specificity, 29% at 85% specificity, 33% at 80% specificity and 35% at 75% specificity.

## Table 5: sensitivity and specificity of log transformed GPC-1 values for differentiating between BPH and CaP samples at different cutpoints.

| | Cut Points | | | |
| | 1.85 | 1.90 | 1.95 | 2.00 |
| --- | --- | --- | --- | --- |
| Sensitivity | 0.300 (0.212-0.400) | 0.390 (0.294-0.493) | **0.510 (0.408-0.611)** | 0.590 (0.487-0.687) |
| Specificity | 0.810 (0.719-0.882) | 0.700 (0.600-0.788) | **0.660 (0.558-0.752)** | 0.570 (0.467-0.669) |
| PPV | 0.612 (0.462-0.748) | 0.565 (0.440-0.684) | **0.600 (0.488-0.705)** | 0.578 (0.477-0.676) |
| NPV | 0.536 (0.454-0.618) | 0.534 (0.445-0.622) | **0.574 (0.478-0.666)** | 0.582 (0.478-0.681) |
| LR+ | 1.58 (0.78-2.38) | 1.30 (0.79-1.81) | **1.50 (1.00-2.01)** | 1.37 (0.99-1.76) |
| LR- | 0.86 (0.73-1.00) | 0.87 (0.69-1.05) | **0.74 (0.56-0.93)** | 0.72 (0.51-0.93) |
| AUC | 0.555 | 0.545 | **0.585** | 0.580 |
| p value* | 0.070 | 0.181 | **0.014** | 0.023 |

| | Cut Points | | | |
| | 2.05 | 2.10 | 2.15 | 2.20 |
| --- | --- | --- | --- | --- |
| Sensitivity | 0.670 (0.569-0.761) | 0.750 (0.653-0.831) | 0.820 (0.731-0.890) | 0.840 (0.753-0.906) |
| Specificity | 0.450 (0.350-0.553) | 0.390 (0.294-0.493) | 0.330 (0.239-0.431) | 0.270 (0.186-0.368) |
| PPV | 0.549 (0.457-0.639) | 0.551 (0.464-0.637) | 0.550 (0.467-0.632) | 0.535 (0.454-0.615) |
| NPV | 0.577 (0.460-0.688) | 0.609 (0.479-0.729) | 0.647 (0.501-0.776) | 0.628 (0.467-0.770) |
| LR+ | 1.22 (0.94-1.49) | 1.23 (0.99-1.47) | 1.22 (1.02-1.43) | 1.15 (0.98-1.32) |
| LR- | 0.73 (0.47-1.00) | 0.64 (0.37-0.91) | 0.55 (0.27-0.82) | 0.59 (0.26-0.92) |
| AUC | 0.560 | 0.570 | 0.575 | 0.550 |
| p value* | 0.081 | 0.033 | 0.014 | 0.057 |

*compared to chance

*Note that a sensitivity or specificity value expressed as "0.30" is equivalent to 30%.*

### Multiple Analyte Analyses

There is an unmet need for prostate cancer tests with high specificity to complement the PSA test's high sensitivity (~80%). Therefore the cutpoint that maximizes specificity was determined. The optimal cutpoint for a high specificity was determined to be 1.85, which gave sensitivity of 0.3 (30%) and a specificity of 0.81 (81%).

The specificity and sensitivity of diagnostic tests can be increased by measuring multiple analytes rather than a single analyte. Therefore the effects of including additional analytes with GPC-1 levels in differentiating BPH and CaP patients were determined.

To maximize differentiation between BPH and CaP patients, a weighted sum optimization procedure using combinations of analytes with different weightings was assessed. A total of 82 different analytes was tested in combination with GPC-1 to optimize performance of the combination. Determination of an optimized weighted sum was performed. The weighted sum was used to establish cut off thresholds whereby the area under the curve (AUC) for the receiver operator curve (ROC) was maximized to minimize the misclassification rate. The sensitivity and specificity were determined at this maximum AUC value. This analysis was independently performed three times.

### - GPC-1 and one other analyte

The results of the AUC optimization analysis for GPC-1 and one other analyte are summarized below in **Table 6**. The frequency of appearance of a particular combination in the three analyses is shown. The following analyte combinations were identified:

**Table 6: AUC optimization analysis for GPC-1 and one other analyte.**

| Unifying Analyte | Additional Analytes | Frequency (out of 3) |
|---|---|---|
| GPC-1 | HGF | 3 |
| | EGF | 3 |
| | PAI-1 | 3 |
| | G-CSF | 3 |
| | PDGFAB.BB | 3 |
| | PDGFBB | 2 |
| | HuIL-18 | 1 |
| | sCD40L | 1 |
| | HuGM-CSF | 1 |
| | HuIFN$\gamma$ | 1 |
| | Follistatin | 1 |

The results show that with the appropriate weighting and analyte combinations, improved AUC, sensitivity and specificity results could be achieved. The AUC values for the top 11 combinations all improved from an AUC of 0.585 for GPC-1 alone. The AUC of the combinations improved between a range of 0.629 (GPC-1/HuGM-CSF, analysis 2) to a maximum of 0.71 (GPC-1/HGF, analysis 3). Sensitivity and/or specificity also showed improvements over GPC-1 alone. For instance, the GPC-1/HGF combination showed sensitivity and specificity of 0.64 and 0.72 compared to 0.51 and 0.66 for GPC-1 alone (analysis 2, **Table 7**).

- GPC-1 *and two analyte analysis*

The optimization analysis was repeated using GPC-1 and two additional analytes. The results of the optimization analyses are shown in **Table 7.**

Table 7: summary of results for sensitivity and specificity of GPC-1 with the addition of one or two analytes.

| GPC-1 analyte combination | Analysis 1 | | | Analysis 2 | | | Analysis 3 | | | | | | | Analysis 4 pAUC | | | | |
|---|---|---|---|---|---|---|---|---|---|---|---|---|---|---|---|---|---|---|
| | AUC | Sens | Spec | AUC | Sens | Spec | AUC | | | Sens at given specificity | | | | AUC | Sens at given specificity | | | |
| | | | | | | | | Sens | Spec | 90% | 85% | 80% | 75% | | 90% | 85% | 80% | 75% |
| GPC-1 | | | | 0.585 | 0.51 | 0.66 | 0.61 | 0.57 | 0.57 | 0.2 | 0.29 | 0.33 | 0.35 | | | | | |
| GPC-1/HGF | 0.7 | 0.65 | 0.65 | 0.703 | 0.64 | 0.72 | 0.71 | 0.64 | 0.64 | 0.37 | 0.45 | 0.51 | 0.54 | 0.7 | 0.39 | 0.41 | 0.52 | 0.56 |
| GPC-1/HGF/PAI-1 | 0.73 | 0.67 | 0.67 | 0.743 | 0.58 | 0.8 | | | | | | | | 0.73 | 0.43 | 0.49 | 0.55 | 0.59 |
| GPC-1/HGF/ HuFGFb | 0.74 | 0.69 | 0.7 | 0.735 | 0.596 | 0.85 | 0.73 | 0.69 | 0.69 | 0.44 | 0.45 | 0.54 | 0.61 | 0.74 | 0.45 | 0.58 | 0.62 | 0.68 |
| GPC-1/HGF/EGF | 0.72 | 0.67 | 0.67 | 0.731 | 0.596 | 0.81 | | | | | | | | 0.71 | 0.32 | 0.36 | 0.52 | 0.61 |
| GPC-1/HGF/ HuPDFGBB | 0.72 | 0.67 | 0.67 | 0.727 | 0.636 | 0.75 | | | | | | | | | | | | |
| GPC-1/HGF/ Follistatin | 0.72 | 0.66 | 0.66 | 0.724 | 0.626 | 0.73 | 0.72 | 0.69 | 0.69 | 0.35 | 0.47 | 0.49 | 0.51 | | | | | |
| GPC-1/HGF/G-CSF | 0.72 | 0.65 | 0 65 | 0.722 | 0.515 | 0.87 | 0.72 | 0.65 | 0.65 | 0.38 | 0.45 | 0.54 | 0.57 | | | | | |
| GPC-1/HGF/sCD-40L | | | | 0.722 | 0.515 | 0.84 | | | | | | | | | | | | |
| GPC-1/HGF/HuIL-8 | | | | 0.721 | 0.545 | 0.85 | | | | | | | | | | | | |
| GPC-1/HGF/ HuCTACK | | | | 0.721 | 0.707 | 0.65 | | | | | | | | | | | | |
| GPC-1/HGF/ PDGFAB.BB | | | | | | | 0.72 | 0.65 | 0.65 | 0.38 | 0.45 | 0.54 | 0.57 | | | | | |
| GPC-1/HGF/IFNg | | | | | | | 0.73 | 0.66 | 0.66 | 0.42 | 0.43 | 0.49 | 0.6 | | | | | |
| GPC-1/HGF/HuM CP-1MCAF | 0.72 | 0.67 | 0.68 | | | | | | | | | | | | | | | |
| GPC-1/HGF/HuIL-18 | 0.72 | 0.68 | 0.68 | | | | | | | | | | | | | | | |
| GPC-1/HGF/HuGM-C5F | 0.73 | 0.67 | 0.67 | | | | | | | | | | | | | | | |
| GPC-1/HGF/uPA | | | | | | | 073 | 0.67 | 0.67 | 0.36 | 0.44 | 0.51 | 0.6 | | | | | |
| GPC-1/HGF/ HuGROa | | | | | | | 0.73 | 0.67 | 0.67 | 0.44 | 0.45 | 0.54 | 0.62 | | | | | |
| GPC-1/HGF/ sVEGFR | | | | | | | 0.72 | 0.64 | 0.64 | 0.44 | 0.47 | 0.49 | 0.53 | | | | | |
| GPC-1/EGF | 0.65 | 0.61 | 0.61 | 0.663 | 0.404 | 0.88 | 0.66 | 0.61 | 0.61 | 0.36 | 0 41 | 0.44 | 0.5 | 0.65 | 0.37 | 0 41 | 0.44 | 0.48 |

(continued)

| GPC-1 analyte combination | Analysis 1 | | | Analysis 2 | | | Analysis 3 | | | | | | | Analysis 4 pAUC | | | | |
|---|---|---|---|---|---|---|---|---|---|---|---|---|---|---|---|---|---|---|
| | AUC | Sens | Spec | AUC | Sens | Spec | AUC | | | Sens at given specificity | | | | AUC | Sens at given specificity | | | |
| | | | | | | | | Sens | Spec | 90% | 85% | 80% | 75% | | 90% | 85% | 80% | 75% |
| GPC-I/EGF/Follistatin | | | | | | | | | | | | | | 0.69 | 0.42 | 0.52 | 0.53 | 0.66 |
| GPC-1/EGF/HGF | 0.72 | 0.67 | 0.67 | 0.731 | 0.596 | 0.81 | | | | | | | | 0.71 | 0.32 | 0.36 | 0.52 | 0.61 |
| GPC-1/EGF/TNFa | | | | | | | | | | | | | | 0.71 | 0.32 | 0.36 | 0.52 | 0.61 |
| GPC-1/PAI-1 | 0.68 | 0.64 | 0.64 | 0.687 | 0.747 | 0.58 | 0.69 | 0.65 | 0.65 | 0.25 | 0.34 | 0 41 | 0.5 | 0.68 | 0.29 | 0.35 | 0.44 | 0.53 |
| GPC-1/PAI-1/HGF | 0.73 | 0.67 | 0.67 | 0.743 | 0.58 | 0.8 | | | | | | | | 0.73 | 0.43 | 0.49 | 0.55 | 0.59 |
| GPC-1/G-CSF | 0.67 | 0.63 | 0.63 | 0.673 | 0.576 | 0.72 | 0.68 | 0.64 | 0.64 | 0.24 | 0.33 | 0.43 | 0.55 | 0.66 | 0.27 | 0.37 | 0.47 | 0.53 |
| GPC-1/G-CSF/HGF | 0.72 | 0.65 | 0.65 | 0.722 | 0.515 | 0.87 | 0.72 | 0.65 | 0.65 | 0.38 | 0.45 | 0.54 | 0.57 | | | | | |
| GPC-1/G-CSF/sTIE2 | | | | 0.721 | 0.677 | 0.74 | | | | | | | | | | | | |
| GPC-1/huL-18 | | | | | | | 0.65 | 0.62 | 0.62 | 0.21 | 0.34 | 0.41 | 0.5 | | | | | |
| GPC-1/huIL-18/HGF | 0.72 | 0.68 | 0.68 | | | | | | | | | | | | | | | |
| GPC-1/PDGFAB.BB | 0.66 | 0.6 | 0.61 | 0.663 | 0.485 | 0.83 | 0.68 | 0.65 | 0.65 | 0.33 | 0.35 | 0.43 | 0.55 | 0.67 | 0.32 | 0.38 | 0.5 | 0.54 |
| GPC-1/PDGDAB.BB/HGF | | | | | | | 0.72 | 0.65 | 0.65 | 0.38 | 0.45 | 0.54 | 0.57 | | | | | |
| GPC-1/PDGFBB | | | | 0.642 | 0.657 | 0.61 | 0.66 | 0.63 | 0.63 | 0.27 | 0.34 | 0.48 | 0.55 | | | | | |
| GPC-1/PDGFBB/HGF | 0.72 | 0.67 | 0.67 | 0.727 | 0.636 | 0.75 | | | | | | | | | | | | |
| GPC-1/sCD40L | | | | | | | 0.65 | 0.6 | 0.6 | 0.29 | 0.36 | 0 41 | 0.5 | | | | | |
| GPC-1/sCD40L/HGF | | | | 0.722 | 0.515 | 0.84 | | | | | | | | | | | | |
| GPC-1/HuGM-CSF | | | | 0.629 | 0.567 | 0.72 | | | | | | | | | | | | |
| GPC-1/HuGM-CSF/HGF | 0.73 | 0.67 | 0.67 | | | | | | | | | | | | | | | |
| GPC-1/HuIFNg | | | | 0.636 | 0.6 | 0.63 | | | | | | | | | | | | |
| GPC-1/HuIFNg/HGF | | | | | | | 0.73 | 0.66 | 0.66 | 0.42 | 0.43 | 0.49 | 0.6 | | | | | |

(continued)

| GPC-1 analyte combination | Analysis 1 | | | Analysis 2 | | | Analysis 3 | | | | | | | Analysis 4 pAUC | | | | |
|---|---|---|---|---|---|---|---|---|---|---|---|---|---|---|---|---|---|---|
| | AUC | Sens | Spec | AUC | Sens | Spec | AUC | | | Sens at given specificity | | | | AUC | Sens at given specificity | | | |
| | | | | | | | | Sens | Spec | 90% | 85% | 80% | 75% | | 90% | 85% | 80% | 75% |
| GPC-1/Fullistatin GPC-1/Follistatin/EGF GPC-1/Follistatin/HGF | 0.72 | 0.66 | 0.66 | 0.724 | 0.626 | 0.73 | 0.65 0.72 | 0.58 0.69 | 0.58 0.69 | 0.26 0.35 | 0.38 0.47 | 0.43 0.49 | 0.44 0.51 | 0.69 | 0.42 | 0.52 | 0.53 | 0.66 |
| | | | | | | | | | | | | | | | | | | |

*The first column (GPC-1 analyte combinations) shows GPC-1 alone or in combinations with one or two other analytes. GPC-1 + 2 analyte combinations are grouped according to the common second analyte. Analyses 1, 2 and 3 show results from three independently performed AUC optimizations. Analysis 3 shows the optimized sensitivity and specificity and also the sensitivity at given fixed specificities (90%, 85%, 80% and 75%). Analysis 4 shows results from a pA UC optimization. The AUC of the resulting curve is shown with the sensitivity at given fixed specificities (90%, 85%, 80% and 75%).*

Addition of two analytes to GPC-1 resulted in further improvements in AUC, sensitivity and/or specificity over either GPC-1 or GPC-1 + 1 analyte (**Table 8**).

**Table 8: addition of two analytes to GPC-1.**

| Analyte(s) | AUC | Sensitivity | Specificity |
|---|---|---|---|
| GPC-1 | 0.585 | 0.51 | 0.62 |
| GPC-1/HGF | 0.703 | 0.64 | 0.72 |
| GPC-1/HGF/FGFb | 0.735 | 0.60 | 0.85 |
| GPC-1/EGF | 0.663 | 0.404 | 0.88 |
| GPC-1/EGF/HGF | 0.731 | 0.60 | 0.81 |

The following GPC-1 plus two analyte combinations were identified as best performing combinations in the analysis (**Table 9**).

**Table 9: preferred GPC-1 plus two analyte combinations.**

| Unifying Analyte | Second Analyte | Third analyte |
|---|---|---|
| GPC-1 | HGF | PAI-1, HuFGFb, EGF, HuPDGFBB, Follistatin, G-CSF, sCD40L, HuIL-8, HuCTACK, PDGFAB.BB, IFNγ, HuMCP-1 MCAF, HuIL-18, HuGM-CSF, uPA, HuGROa or sVEGFR |
| | EGF | Follistatin, HGF, TNFα |
| | PAI-1 | HGF |
| | G-CSF | HGF, sTIE2 |
| | HuIL-18 | HGF |
| | PDGFAB.BB | HGF |
| | PDGFBB | HGF |
| | sCD40L | HGF |
| | HuGM-CSF | HGF |
| | Follistatin | EGF, HGF |

*- pAUC optimization*

The results from the AUC optimization analysis indicated that use of GPC-1 plus one or two analytes could improve the discrimination between Arm 2 BPH and Arm 3 CaP compared to GPC-1 as one of the analytes.

The aim of identifying optimal combinations of GPC-1 and one or two analytes was to maximize the specificity of discriminating between BPH and CaP patient groups.

A revised optimization approach was developed whereby a weighted sum Nelder-Mead optimization procedure was applied to optimize for a partial AUC. pAUC represents the area under the ROC within a restricted (1 -specificity) range, and generally this range lies between values 0 and e, where e must be specified. Here e was set to 0.5. A ROC that rises earlier than another ROC that rises later, when both AUCs are equal, will have higher specificity. Optimizing for pAUC rather than the full AUC optimizes for tests with higher specificity.

The partial AUC optimization procedure was applied to GPC-1 + one (**Table 10**) and GPC-1 + two analyte approaches (**Table 11**). Sensitivities at fixed Specificities of 90%, 85%, 80% and 75% were determined. Results are shown as "Analysis 4", **Table 7**.

The partial AUC optimization procedure for GPC-1 plus one additional analyte was performed. As was observed for the AUC optimization, incorporation of one analyte in addition to GPC-1 improved the discrimination between BPH and CaP (**Table 7** and **Table 12** below). The top 5 analytes identified present in the GPC-1 + 1 analyte combinations were as follows:

**Table 10: GPC-1 plus one analyte combinations used in partial AUC optimization.**

| Unifying Analyte | Additional Analytes |
|---|---|
| GPC-1 | HGF |
| | PAI-1 |
| | G-CSF |
| | PDGFAB.BB |
| | HuIL-9 |

With the exception of HuIL-9, all these analytes were in common with those identified using the AUC weighted sum optimization procedure described previously herein.

The pAUC optimization was performed using GPC-1 plus two additional analytes. The top performing GPC-1 plus two analyte combinations were as follows:

**Table 11: GPC-1 plus two analyte combinations used in partial AUC optimization.**

| Unifying Analyte | Second Analytes | Third analyte |
|---|---|---|
| **GPC-1** | HGF | HuFGFb, PAI-1, EGF |
| | EGF | Follistatin, HGF, TNFα |

Addition of a third analyte improved sensitivity at a given fixed specificity (Table 12).

**Table 12: AUC and sensitivity at fixed specificities for GPC-1 and GPC-1 analyte combinations derived from pAUC optimization.**

| | | Sensitivity at fixed Specificity | | | |
|---|---|---|---|---|---|
| Analyte(s) | AUC | 90% | 85% | 80% | 75% |
| GPC-1 | 0.61 | 0.2 | 0.29 | 0.33 | 0.35 |
| GPC-1/HGF | 0.7 | 0.39 | 0.41 | 0.52 | 0.56 |
| GPC-1/HGF/FGFb | 0.74 | 0.45 | 0.58 | 0.62 | 0.68 |
| GPC-1/EGF | 0.663 | 0.37 | 0.41 | 0.44 | 0.48 |
| GPC-1/EGF/HGF | 0.731 | 0.42 | 0.54 | 0.54 | 0.62 |

*- Best performing combinations to differentiate BPH and CaP*

The results of the 4 separate analyses (3 AUC optimizations and 1 pAUC optimizations) indicated that the following combinations occurred most frequently and provided the best discrimination (**Table 13**).

**Table 13: combinations with highest frequency and optimal discrimination capacity.**

| Combination | Freq | Best AUC | Best SENS/Spec | Best Sens/SPEC |
|---|---|---|---|---|
| GPC-1/HGF | 4/4 | 0.71 | 0.65/0.65 | 0.45/0.85 |
| GPC-1/PAI-1 | 4/4 | 0.69 | 0.75/0.58 | 0.35/0.85 |
| GPC-1/G-CSF | 4/4 | 0.68 | 0.64/0.64 | 0.37/0.85 |
| GPC-1/EGF | 4/4 | 0.66 | 0.61/0.61 | 0.40/0.88 |
| GPC-1/PDGFAB.BB | 4/4 | 0.68 | 0.65/0.65 | 0.49/0.83 |
| GPC-1/HGF/FGFb | 4/4 | 0.74 | 0.69/0.70 | 0.60/0.85 |
| GPC-1/HGF/PAI-1 | 3/4 | 0.74 | 0.67/0.67 | 0.58/0.80 |
| GPC-1/HGF/EGF | 3/4 | 0.73 | 0.67/0.67 | 0.60/0.81 |
| GPC-1/HGF/Follistatin | 3/4 | 0.72 | 0.69/0.69 | 0.47/0.85 |

(continued)

| Combination | Freq | Best AUC | Best SENS/Spec | Best Sens/SPEC |
|---|---|---|---|---|
| GPC-1/HGF/G-CSF | 3/4 | 0.72 | 0.65/0.66 | 0.52/0.87 |
| **Note** : SENS/Spec means the combination of sensitivity and specificity with the highest sensitivity **Note** : Sens/SPEC means the combination of sensitivity and specificity with the highest specificity | | | | |

**Cross-Reference**

The present invention claims priority from Australian provisional patent application number 2015902919 filed on 22 July 2015 entitled "Biomarker combinations for prostate disease" and filed in the name of Minomic International Ltd,

**Claims**

1. A method for determining whether a subject has benign prostatic hyperplasia (BPH) from prostate cancer (CaP), comprising:

    (a) generating a threshold value for discerning between BPH and prostate cancer by:

        detecting at least two analytes in a series of biological samples obtained from a population of subjects having BPH and a population of subjects having CaP, to thereby obtain an analyte level for each said analyte in each said biological sample of the series;
        combining the analyte levels in a manner that allows discrimination between the BPH and CaP samples; and selecting a threshold value from the combined analyte levels and using said threshold value as a basis to discriminate between BPH and CaP in the sample; and

    (b) detecting said at least two analytes in a biological sample from a subject to thereby obtain an analyte level for each said analyte in the subject's biological sample; and
    (c) applying a suitable algorithm and/or transformation to the individual or combined analyte levels obtained from the subject's biological sample to thereby generate a patient analyte value for comparison to the threshold value; and
    (d) determining whether the subject has BPH or CaP by comparison of the patient analyte value to the threshold value,
    wherein the at least two analytes comprise glypican-1 (GPC-1) and are selected from any one of the following analyte combinations:

        GPC-1 and hepatocyte growth factor (HGF)
        GPC-1 and epidermal growth factor (EGF);
        GPC-1 and plasminogen activator inhibitor 1 (PAI-1);
        GPC-1 and granulocyte colony stimulating factor (G-CSF);
        GPC-1 and human Interleukin 18 (HuIL-18);
        GPC-1 and platelet derived growth factor AB.BB (PDGFAB.BB);
        GPC-1 and platelet derived growth factor BB (PDGFBB);
        GPC-1 and soluble CD40 Ligand (sCD40L);
        GPC-1 and human granulocyte macrophage colony stimulating factor (HuGM-CSF);
        GPC-1 and interferon gamma (IFNγ); and
        GPC-1 and follistatin.

2. The method according to claim 1, wherein the at least two analytes are selected from any one of the following analyte combinations:

        GPC-1, HGF and PAI-1;
        GPC-1, HGF and human fibroblast growth factor beta (HuFGFb);
        GPC-1, HGF and EGF;
        GPC-1, HGF and PDGFBB;
        GPC-1, HGF and follistatin;

GPC-1, HGF and G-CSF;
GPC-1, HGF and human interleukin 8 (HuIL-8);
GPC-1, HGF and soluble CD40 ligand (sCD40L);
GPC-1, HGF and human cutaneous T-cell attracting cytokine (CTACK - also known as C-C motif ligand 27 or CCL27);
GPC-1, HGF and PDGFAB.BB;
GPC-1, HGF and IFN$\gamma$;
GPC-1, HGF and human monocyte chemotactic and activating factor (HuMCPI/MCAF);
GPC-1, HGF, and HuIL-18;
GPC-1, HGF, HuGM-CSF; GPC-1, HGF, and urokinase plasminogen activator (uPA);
GPC-1, HGF, and human melanoma growth stimulating activity alpha (HuGROa, also known as CXC motif ligand 1, CXCL1);
GPC-1, HGF and soluble vascular endothelial growth factor receptor (sVEGFR);
GPC-1, EGF and follistatin;
GPC-1, EGF and tumour necrosis factor alpha (TNF$\alpha$); and
GPC-1, G-CSF and soluble tyrosine kinase with immunoglobulin like and EGF-like domains 2 (sTIE2).

3. The method of claim 1 or claim 2, wherein:

(i) said selecting the threshold value from the combined analyte levels comprises selecting a subset of the combined analyte levels; and/or
(ii) said combining of the analyte levels in a manner that allows discrimination between the BPH and CaP samples comprises combining a weighted linear sum of these analyte levels in a manner that maximizes the discrimination between the BPH and CaP samples in accordance with the formula:

$$S = w_1A_1 + w_2A_2 \ldots w_nA_n$$

wherein S is the weighted sum,
w is the final analyte weighting,

and A is the level of a given analyte from n different analytes, or a transformation of the level of a given analyte either numerically or as a ratio of analyte values.

4. The method of claim 3, wherein:

(i) said transformation of the level of a given analyte numerically comprises use of an exponential function, a power function and/or a root function; and/or
(ii) obtaining the final analyte weightings w comprises generating initial analyte weightings (W) that are set to 1, or generated according to the formula:

$$W = \max(M)/M, \text{ where } W = \{w_1, \ldots w_n\} \text{ and } M = \{\text{median}(A_1), \ldots \text{median}(A_n)\};$$

wherein max (M) is a maximum median level of the analyte levels obtained, and M is a vector containing the median analyte level obtained for each said analyte.

5. The method according to claim 4, wherein each said final weight is determined using a discriminating function for optimization in discerning between BPH and CaP in the series of biological samples.

6. The method according to claim 5, wherein:

(a) the discriminating function is any one or more of:

(i) the area under a curve generated by a ROC analysis;
(ii) a combination of True Positive Rates (TPR) and True Negative Rates (TNR);
(iii) the area under a curve generated by a ROC analysis within a restricted specificity range;
(iv) the area under a curve generated by a ROC analysis within a restricted sensitivity range; and/or

(b) the discriminating function for optimization is any one or more of the Nelder-Mead (simplex method), a stochastic method, a gradient descent method, a stochastic gradient descent method, a genetic algorithm, a particle-swarm method, and/or a brute force method.

7. The method according to any one of claims 1 to 6, wherein:

(i) the suitable algorithm and/or transformation applied to the individual or combined analyte levels obtained from the subject's biological sample is in accordance with the formula:

$$S = w_1A_1 + w_2A_2 \ldots w_nA_n$$

wherein S is the weighted sum,
w is the final analyte weighting,
and A is the level of a given analyte from n different analytes, or a transformation of the level of a given analyte either numerically or as a ratio of analyte values; and/or

(ii) said combining the analyte levels maximizes discrimination between the BPH and CaP samples.

8. The method of any one of claims 1 to 7, comprising selecting the threshold value from the combined analyte levels in a manner that:

(i) reduces the misclassification rate between BPH and CaP; and/or
(ii) increases sensitivity in discriminating between BPH and CaP; and/or
(iii) increases specificity in discriminating between BPH and CaP.

9. The method of claim 8, wherein said selecting the threshold value from the combined analyte levels in a manner that reduces the misclassification rate between BPH and CaP comprises any one or more of:

(i) selecting a suitable true positive and/or true negative rate;
(ii) minimizing the misclassification rate;
(iii) minimizing the misclassification rate between BPH and CaP by identifying a point where the true positive rate intersects the true negative rate.

10. The method according to claim 8, wherein:

(i) selecting the threshold value from the combined analyte levels in a manner that increases sensitivity in discriminating between BPH and CaP comprises using a weighted sum Nelder-Mead optimization procedure to optimize for a partial area under a curve generated by a ROC analysis (pAUC), wherein the pAUC represents the area under the ROC within a restricted (1- sensitivity) range that is between value 0 and a specified value e (e.g. 0.5); and or
(ii) said selecting the threshold value from the combined analyte levels in a manner that increases sensitivity in discriminating between BPH and CaP maximizes said sensitivity, or minimizes said specificity.

11. The method according to claim 8 or claim 10, wherein selecting the threshold value from the combined analyte levels in a manner that increases specificity in discriminating between BPH and CaP comprises using a weighted sum Nelder-Mead optimization procedure to optimize for a partial area under a curve generated by a ROC analysis (pAUC), wherein the pAUC represents the area under the ROC within a restricted (1- specificity) range that is between values 0 and a specified value e (e.g. 0.5).

12. The method according to any one of claims 8, 10 or 11, wherein the at least two analytes are selected from any one of the following analyte combinations:

GPC-1/EGF/follistatin;
GPC-1/EGF/TNFα; GPC-1/HGF;
GPC-1/HGF/PAI-1;
GPC-1/HGF/HuFGFb;
GPC-1/HGF/EGF;

GPC-1/HGF/HuPDFGBB;
GPC-1/EGF;
GPC-1/PAI-1;
GPC-1/G-CSF; and
GPC-1/PDGFAB.BB.

**13.** The method according to any one of claims 1 to 12, wherein the at least two analytes are selected from any one of the following analyte combinations:

GPC-1/HGF;
GPC-1/PAI-1;
GPC-1/G-CSF;
GPC-1/EGF;
GPC-1/PDGFAB.BB;
GPC-1/HGF/FGFb;
GPC-1/HGF/PAI-1;
GPC-1/HGF/EGF;
GPC-1/HGF/Follistatin; and
GPC-1/HGF/G-CSF.

**14.** The method according to any one of claims 1 to 13, wherein said detecting at least two analytes in the subject's biological sample comprises:

(i) measuring one or more fluorescent signals indicative of each said analyte level;
(ii) obtaining a measurement of weight/volume of said analytes in the samples;
(iii) measuring an absorbance signal indicative of each said analyte level; or
(iv) using a technique selected from the group consisting of: mass spectrometry, a protein array technique, high performance liquid chromatography (HPLC), gel electrophoresis, radiolabeling, and any combination thereof.

**15.** The method according to any one of claims 1 to 14, wherein:

(i) said obtaining an analyte level in the biological samples of the series and the subject's biological sample comprises contacting each said sample with first and second antibody populations, wherein each said antibody population has binding specificity for one of said analytes, and the first and second antibody populations have different binding specificities; and/or
(ii) the biological samples of the series and the subject's biological sample are each whole blood, serum, plasma, saliva, tear/s, urine, or tissue.

**Patentansprüche**

**1.** Ein Verfahren zur Bestimmung, ob ein Patient an benigner Prostatahyperplasie (BPH) durch Prostatakrebs (CaP) leidet, umfassend:

(a) Generieren eines Schwellenwertes zur Unterscheidung zwischen BPH und Prostatakrebs durch:

Nachweis von mindestens zwei Analyten in einer Reihe von biologischen Proben, die aus einer Population von Personen mit BPH und einer Population von Personen mit CaP erhalten wurden, um dadurch einen Analytspiegel für jeden Analyten in jeder biologischen Probe der Reihe zu erhalten;
Kombinieren der Analytkonzentrationen in einer Weise, die eine Unterscheidung zwischen den BPH- und CaP-Proben ermöglicht; und
Auswahl eines Schwellenwertes aus den kombinierten Analytwerten und Verwendung dieses Schwellenwertes als Grundlage zur Unterscheidung zwischen BPH und CaP in der Probe; und

(b) Nachweisen der mindestens zwei Analyten in einer biologischen Probe von einem Proband, um dadurch ein Analytgehalt für jeden der Analyten in der biologischen Probe des Probanden zu erhalten; und
(c) Anwendung eines geeigneten Algorithmus und/oder einer Transformation auf die einzelnen oder kombinierten Analytpegel, die aus der biologischen Probe des Probanden erhalten wurden, um dadurch einen Patienten-Analytwert zum Vergleich mit dem Schwellenwert zu erzeugen; und

(d) Bestimmung, ob der Proband BPH oder CaP hat, durch Vergleich des Patienten-Analytwertes mit dem Schwellenwert,

wobei die mindestens zwei Analyten Glypican-1 (GPC-1) umfassen und aus einer der folgenden Analytkombinationen ausgewählt sind:

GPC-1 und Hepatozyten-Wachstumsfaktor (HGF)

GPC-1 und epidermaler Wachstumsfaktor (EGF);

GPC-1 und Plasminogenaktivator-Inhibitor 1 (PAI-1);

GPC-1 und Granulozyten-Kolonie-stimulierender Faktor (G-CSF);

GPC-1 und menschliches Interleukin 18 (HuIL-18);

GPC-1 und von Blutplättchen abgeleiteter Wachstumsfaktor AB.BB (PDGFAB.BB);

GPC-1 und von Blutplättchen abgeleiteter Wachstumsfaktor BB (PDGFBB);

GPC-1 und löslicher CD40-Ligand (sCD40L);

GPC-1 und menschlicher Granulozyten-Makrophagen-Kolonie-stimulierender Faktor (HuGM-CSF);

GPC-1 und Interferon-Gamma (IFNy); und

GPC-1 und Follistatin.

2. Das Verfahren nach Anspruch 1, wobei die mindestens zwei Analyten aus einer der folgenden Analytkombinationen ausgewählt werden:

GPC-1, HGF und PAI-1;

GPC-1, HGF und humaner Fibroblasten-Wachstumsfaktor beta (HuFGFb);

GPC-1, HGF und EGF;

GPC-1, HGF und PDGFFBB;

GPC-1, HGF und Follistatin;

GPC-1, HGF und G-CSF;

GPC-1, HGF und menschliches Interleukin 8 (HuIL-8);

GPC-1, HGF und löslicher CD40-Ligand (sCD40L);

GPC-1, HGF und humanes kutanes T-Zell-anziehendes Zytokin (CTACK - auch bekannt als C-C-Motiv Ligand 27 oder CCL27);

GPC-1, HGF und PDGFAB.BB;

GPC-1, HGF und IFN$\gamma$;

GPC-1, HGF und chemotaktischer und aktivierender Faktor für menschliche Monozyten (HuMCP1/MCAF);

GPC-1, HGF und HuIL-18;

GPC-1, HGF, HuGM-CSF; GPC-1, HGF und Urokinase-Plasminogenaktivator (uPA);

GPC-1, HGF und das Wachstum des menschlichen Melanoms stimulierende Aktivität Alpha (HuGROa, auch bekannt als CXC-Motivligand 1, CXCL1);

GPC-1, HGF und löslicher vaskulärer endothelialer Wachstumsfaktor-Rezeptor (sVEGFR);

GPC-1, EGF und Follistatin;

GPC-1, EGF und Tumornekrosefaktor alpha (TNFa); und

GPC-1, G-CSF und lösliche Tyrosinkinase mit immunglobulinähnlichen und EGF-ähnlichen Bereiche 2 (sTIE2).

3. Das Verfahren nach Anspruch 1 oder Anspruch 2, wobei:

(i) wobei das Auswählen des Schwellenwertes aus den kombinierten Analytgehalten das Auswählen einer Teilmenge der kombinierten Analytgehalte umfasst; und/oder

(ii) wobei das Kombinieren der Analytgehalte in einer Weise, die die Unterscheidung zwischen den BPH- und CaP-Proben ermöglicht, das Kombinieren einer gewichteten linearen Summe dieser Analytgehalte in einer Weise umfasst, die die Unterscheidung zwischen den BPH- und CaP-Proben gemäß der Formel maximiert:

$$S = w_1A_1 + w_2A_2 ... w_nA_n$$

wobei S die gewichtete Summe ist,

w ist die endgültige Analyt-Gewichtung,

und A ist das Niveau eines bestimmten Analyten aus n verschiedenen Analyten oder eine Transformation des Gehalts eines bestimmten Analyten entweder numerisch oder als Verhältnis der Analytgehalte.

4. Das Verfahren nach Anspruch 3, wobei:

(i) die Transformation des Gehalts eines gegebenen Analyten numerisch die Verwendung einer Exponentialfunktion, einer Potenzfunktion und/oder einer Wurzelfunktion umfasst; und/oder
(ii) Das Erhalten der endgültigen Analyt-Gewichtungen w umfasst das Erzeugen von anfänglichen Analyt-Gewichtungen (W), die auf 1 gesetzt oder gemäß der Formel erzeugt werden:

$$W = \max{(M)}/M, \text{ wobei } W = \{w_1, ...w_n\} \text{ und } M = \{\text{Median}(A_1), ...\text{Median}(A_n)\};$$

wobei max (M) ein maximaler Medianwert der erhaltenen Analytgehalte ist, und M ein Vektor ist, der den für jeden der genannten Analyte erhaltenen Medianwert der Analytgehalt enthält.

5. das Verfahren nach Anspruch 4, bei dem jedes Endgewicht unter Verwendung einer Unterscheidungsfunktion zur Optimierung bei der Unterscheidung zwischen BPH und CaP in der Serie biologischer Proben bestimmt wird.

6. Das Verfahren nach Anspruch 5, wobei:

(a) die diskriminierende Funktion eine oder mehrere ist:

(i) die Fläche unter einer durch eine ROC-Analyse erzeugten Kurve;
(ii) eine Kombination aus wahr-positiven Raten (TPR) und wahr-negativen Raten (TNR);
(iii) die Fläche unter einer Kurve, die durch eine ROC-Analyse innerhalb eines begrenzten Spezifizitätsbereichs erzeugt wird;
(iv) die Fläche unter einer Kurve, die durch eine ROC-Analyse innerhalb eines eingeschränkten Empfindlichkeitsbereichs erzeugt wird; und/oder

(b) die Unterscheidungsfunktion für die Optimierung eine oder mehrere der folgenden Methoden ist: Nelder-Mead (Simplex-Methode), eine stochastische Methode, eine Gradientenabstiegsmethode, eine stochastische Gradientenabstiegsmethode, ein genetischer Algorithmus, eine Partikel-Schwarm-Methode und/oder eine Brute-Force-Methode.

7. Das Verfahren nach einem der Ansprüche 1 bis 6, wobei

(i) der geeignete Algorithmus und/oder die geeignete Transformation, die auf die einzelnen oder kombinierten Analytgehalte angewandt werden, die aus der biologischen Probe des Probanden gewonnen werden, der Formel entsprechen:

$$S = w_1 A_1 + w_2 A_2 ... w_n A_n$$

wobei S die gewichtete Summe ist,
w ist die endgültige Analyt-Gewichtung,
und A das Niveau eines bestimmten Analyten aus n verschiedenen Analyten oder eine Transformation des Gehalts eines bestimmten Analyten entweder numerisch oder als Verhältnis der Analytwerte ist; und/oder

(ii) sagte, dass die Kombination der Analytkonzentrationen die Diskriminierung zwischen den BPH- und CaP-Proben maximiert.

8. Das Verfahren nach einem der Ansprüche 1 bis 7, die darin besteht, den Schwellenwert aus den kombinierten Analytgehalten in einer Weise auszuwählen, dass

(i) reduziert die Fehlklassifikationsrate zwischen BPH und CaP; und/oder
(ii) die Sensibilität bei der Unterscheidung zwischen BPH und CaP erhöht; und/oder
(iii) erhöht die Spezifität bei der Unterscheidung zwischen BPH und CaP.

9. Das Verfahren nach Anspruch 8, bei dem die Auswahl des Schwellenwertes aus den kombinierten Analytkonzentrationen in einer Weise erfolgt, die die Fehlklassifizierungsrate zwischen BPH und CaP verringert, umfasst eines oder mehrere der folgenden Verfahren:

(i) Auswahl einer geeigneten wahr-positiven und/oder wahr-negativen Rate;
(ii) Minimierung der Fehlklassifikationsrate;
(iii) Minimierung der Fehlklassifizierungsrate zwischen BPH und CaP durch Identifizierung eines Punktes, an dem sich die wahr-positive Rate mit der wahr-negativen Rate schneidet.

10. Das Verfahren nach Anspruch 8, wobei:

(i) Auswählen des Schwellenwertes aus den kombinierten Analytgehalten in einer Weise, die die Empfindlichkeit bei der Unterscheidung zwischen BPH und CaP erhöht, umfassend die Verwendung eines Nelder-Mead-Optimierungsverfahrens mit gewichteter Summe zur Optimierung für eine Teilfläche unter einer durch eine ROC-Analyse (pAUC) erzeugten Kurve, wobei die pAUC die Fläche unter der ROC innerhalb eines eingeschränkten (1-Empfindlichkeits-)Bereichs darstellt, der zwischen dem Wert 0 und einem spezifizierten Wert e (z.B. 0,5) liegt; und oder
(ii) wobei die Auswahl des Schwellenwertes aus den kombinierten Analytgehalten in einer Weise erfolgt, die die Sensitivität bei der Unterscheidung zwischen BPH und CaP erhöht und die Sensitivität maximiert oder die Spezifität minimiert.

11. Das Verfahren nach Anspruch 8 oder 10, wobei das Auswählen des Schwellenwertes aus den kombinierten Analytgehalten in einer Weise, die die Spezifität bei der Unterscheidung zwischen BPH und CaP erhöht, die Verwendung eines Nelder-Mead-Optimierungsverfahrens mit gewichteter Summe umfasst, um für eine Teilfläche unter einer durch eine ROC-Analyse (pAUC) erzeugten Kurve zu optimieren, wobei die pAUC die Fläche unter der ROC innerhalb eines eingeschränkten Bereichs (1-Spezifität) darstellt, der zwischen den Werten 0 und einem spezifizierten Wert e (z.B. 0,5) liegt.

12. Das Verfahren nach einem der Ansprüche 8, 10 oder 11, wobei die mindestens zwei Analyten aus einer der folgenden Analytkombinationen ausgewählt werden:

GPC-1/EGF/Follistatin;
GPC-1/EGF/TNF$\alpha$; GPC-1/HGF;
GPC-1/HGF/PAI-1;
GPC-1/HGF/HuFGFb;
GPC-1/HGF/EGF;
GPC-1/HGF /HuPDFGBB;
GPC-1/EGF;
GPC-1/PAI-1;
GPC-1/G-CSF; und
GPC-1/PDGFAB.BB.

13. Das Verfahren nach einem der Ansprüche 1 bis 12, wobei die mindestens zwei Analyten aus einer der folgenden Analytkombinationen ausgewählt werden:

GPC-1/HGF;
GPC-1/PAI-1;
GPC-1/G-CSF;
GPC-1/EGF;
GPC-1/PDGFAB.BB;
GPC-1/HGF/FGFb;
GPC-1/HGF/PAI-1;
GPC-1/HGF/EGF;
GPC-1/HGF/Follistatin; und
GPC-1/HGF /G-CSF.

14. Das Verfahren nach einem der Ansprüche 1 bis 13, wobei der Nachweis von mindestens zwei Analyten in der biologischen Probe des Probanden umfasst:

(i) Messung eines oder mehrerer Fluoreszenzsignale, die für jeden der genannten Analytwerte kennzeichnend sind;
(ii) Erhalten einer Messung von Gewicht/Volumen der genannten Analyten in den Proben;
(iii) Messen eines Absorptionssignals, das für jeden der genannten Analytgehalte kennzeichnend ist; oder
(iv) unter Verwendung einer Technik, ausgewählt aus der Gruppe bestehend aus: Massenspektrometrie, einer Protein-Array-Technik, Hochleistungs-Flüssigkeitschromatographie (HPLC), Gelelektrophorese, Radiomarkierung und jeder Kombination davon.

**15.** die Methode nach einem der Ansprüche 1 bis 14, wobei

(i) wobei das Erhalten eines Analytgehalts in den biologischen Proben der Serie und der biologischen Probe des Probanden das Inkontaktbringen jeder Probe mit einer ersten und einer zweiten Antikörperpopulation umfasst, wobei jede Antikörperpopulation Bindungsspezifität für einen der Analyten aufweist und die erste und die zweite Antikörperpopulation unterschiedliche Bindungsspezifitäten aufweisen; und/oder
(ii)bei den biologischen Proben der Serie und der biologischen Probe des Probanden handelt es sich jeweils um Vollblut, Serum, Plasma, Speichel, Tränenflüssigkeit(en), Urin oder Gewebe.

**Revendications**

**1.** Procédé de détermination du fait qu'un sujet souffre d'hyperplasie bénigne de la prostate (BPH) ou du cancer de la prostate (CaP), comprenant :

(a) la génération d'une valeur seuil pour différencier la BPH du cancer de la prostate par :

détection d'au moins deux analytes dans une série d'échantillons biologiques obtenus à partir d'une population de sujets souffrant de BPH et d'une population de sujets présentant un CaP, pour ainsi obtenir un niveau d'analytes pour chacun desdits analytes dans chacun desdits échantillons biologiques de la série ; combinaison des niveaux d'analytes d'une manière qui permet la discrimination entre les échantillons de BPH et de CaP ; et
sélection d'une valeur seuil à partir des niveaux d'analytes combinés et utilisation de ladite valeur seuil en tant que base pour différencier la BPH du CaP dans l'échantillon ; et

(b) la détection desdits au moins deux analytes dans un échantillon biologique d'un sujet pour ainsi obtenir un niveau d'analyte pour chacun desdits analytes dans l'échantillon biologique du sujet ; et
(c) l'application d'un algorithme et/ou d'une transformation adaptés aux niveaux d'analytes individuels ou combinés obtenus de l'échantillon biologique du sujet pour ainsi générer une valeur d'analyte patient pour la comparaison à la valeur seuil ; et
(d) la détermination du fait que le sujet souffre de BPH ou de CaP par comparaison de la valeur d'analyte patient à la valeur seuil,
dans lequel les au moins deux analytes comprennent le glypican-1 (GPC-1) et sont sélectionnés parmi l'une quelconque des combinaisons d'analytes suivantes :

GPC-1 et le facteur de croissance des hépatocytes (HGF) ;
GPC-1 et le facteur de croissance épidermique (EGF) ;
GPC-1 et l'inhibiteur de l'activateur du plasminogène 1 (PAI-1) ;
GPC-1 et le facteur de stimulation des colonies de granulocytes (G-CSF) ;
GPC-1 et l'interleukine 18 humaine (HuIL-18) ;
GPC-1 et le facteur de croissance dérivé des plaquettes AB.BB (PDGFAB.BB) ;
GPC-1 et le facteur de croissance dérivé des plaquettes BB (PDGFBB) ;
GPC-1 et le ligand CD40 soluble (sCD40L) ;
GPC-1 et le facteur de stimulation des colonies de granulocytes et de macrophages humain (HuGM-CSF) ;
GPC-1 et l'interféron gamma (IFNγ) ; et
GPC-1 et la follistatine.

**2.** Procédé selon la revendication 1, dans lequel les au moins deux analytes sont sélectionnés parmi l'une quelconque des combinaisons d'analytes suivantes :

GPC-1, HGF et PAI-1 ;
GPC-1, HGF et facteur de croissance des fibroblastes β humain (HuFGFb) ;
GPC-1, HGF et EGF ;
GPC-1, HGF et PDGFBB ;
GPC-1, HGF et follistatine ;
GPC-1, HGF et G-CSF ;
GPC-1, HGF et interleukine 8 humaine (HuIL-8) ;
GPC-1, HGF et ligand CD40 soluble (sCD40L) ;
GPC-1, HGF et cytokine attirant les lymphocytes T cutanés humaine (CTACK - également connue comme le ligand 27 à motif C-C ou CCL27) ;
GPC-1, HGF et PDGFAB.BB ;
GPC-1, HGF et IFNγ ;
GPC-1, HGF et facteur d'activation et chimiotactique des monocytes humain (HuMCP1/MCAF) ;
GPC-1, HGF, et HuIL-18 ;
GPC-1, HGF, HuGM-CSF ;
GPC-1, HGF, et activateur du plasminogène urokinase (uPA) ;
GPC-1, HGF, et activité de stimulation de croissance des mélanomes α humaine (HuGROa, également connue comme le ligand 1 à motif CXC, CXCL1) ;
GPC-1, HGF et récepteur du facteur de croissance endothélial vasculaire soluble (sVEGFR) ;
GPC-1, EGF et follistatine ;
GPC-1, EGF et facteur de nécrose des tumeurs α (TNFα) ; et
GPC-1, G-CSF et TIE2 soluble (soluble tyrosine kinase with immunoglobulin like and EGF-like domains 2).

3. Procédé selon la revendication 1 ou la revendication 2, dans lequel :

(i) ladite sélection de la valeur seuil à partir des niveaux d'analytes combinés comprend la sélection d'un sous-ensemble des niveaux d'analytes combinés ; et/ou
(ii) ladite combinaison des niveaux d'analytes d'une manière qui permet la discrimination entre les échantillons de BPH et de CaP comprend la combinaison d'une somme linéaire pondérée de ces niveaux d'analytes d'une manière qui maximise la discrimination entre les échantillons de BPH et de CaP conformément à la formule :

$$S = w_1 A_1 + w_2 A_2 \ldots w_n A_n$$

dans laquelle S est la somme pondérée,
w est la pondération finale de l'analyte,

et A est le niveau d'un analyte donné parmi n analytes différents, ou une transformation du niveau d'un analyte donné soit numériquement soit comme un rapport de valeurs d'analytes.

4. Procédé selon la revendication 3, dans lequel

(i) ladite transformation du niveau d'un analyte donné numériquement comprend l'utilisation d'une fonction exponentielle, d'une fonction de puissance et/ou d'une fonction de racine ; et/ou
(ii) l'obtention des pondérations d'analytes finales w comprend la génération de pondérations d'analytes initiales (W) qui sont définies à 1, ou générées selon la formule :

$$W = \max(M)/M, \text{ où } W = \{w_1, \ldots w_n\} \text{ et } M = \{(A_1)\text{médian}, \ldots (A_n)\text{médian}\} ;$$

où max(M) est un niveau médian maximal des niveaux d'analytes obtenus, et M est un vecteur contenant le niveau médian d'analytes obtenu pour chacun desdits analytes.

5. Procédé selon la revendication 4, dans lequel chacun desdits poids finaux est déterminé en utilisant une fonction de discrimination pour l'optimisation dans la différenciation entre la BPH et le CaP dans la série des échantillons biologiques.

6. Procédé selon la revendication 5, dans lequel :

(a) la fonction de discrimination est l'une quelconque ou plus de :

(i) l'aire sous une courbe générée par une analyse ROC ;
(ii) une combinaison de taux de vrais positifs (TPR) et de taux de vrais négatifs (TNR) ;
(iii) l'aire sous la courbe générée pas une analyse ROC dans une plage de spécificité restreinte ;
(iv) l'aire sous une courbe générée par une analyse ROC dans une plage de sensibilité restreinte ; et/ou

(b) la fonction de discrimination pour l'optimisation est l'une quelconque ou plus de la méthode Nelder-Mead Simplex, d'une méthode stochastique, d'une méthode de descente de gradient, d'une méthode de descente de gradient stochastique, d'un algorithme génétique, d'une méthode par essaim de particules, et/ou d'une méthode force brute.

7. Procédé selon l'une quelconque des revendications 1 à 6, dans lequel :

(i) l'algorithme et/ou la transformation adaptés appliqués aux niveaux d'analytes individuels ou combinés obtenus de l'échantillon biologique du sujet sont conformes à la formule :

$$S = w_1 A_1 + w_2 A_2 \ldots w_n A_n$$

dans laquelle S est la somme pondérée,
w est la pondération finale de l'analyte,
et A est le niveau d'un analyte donné parmi n analytes différents, ou une transformation du niveau d'un analyte donné soit numériquement soit comme un rapport de valeurs d'analytes ; et/ou

(ii) ladite combinaison des niveaux d'analytes maximise la discrimination entre les échantillons de BPH et de CaP.

8. Procédé selon l'une quelconque des revendications 1 à 7, comprenant la sélection de la valeur seuil à partir des niveaux d'analytes combinés d'une manière qui :

(i) réduit le taux de mauvaise classification entre la BPH et le CaP ; et/ou
(ii) augmente la sensibilité de discrimination entre la BPH et le CaP ; et/ou
(iii) augmente la spécificité de discrimination entre la BPH et le CaP.

9. Procédé selon la revendication 8, dans lequel ladite sélection de la valeur seuil à partir des niveaux d'analytes combinés d'une manière qui réduit le taux de mauvaise classification entre la BPH et le CaP comprend l'un quelconque ou plus de :

(i) la sélection d'un taux de vrais positifs et/ou de vrais négatifs adapté ;
(ii) la minimisation du taux de mauvaise classification ;
(iii) la minimisation du taux de mauvaise classification entre la BPH et le CaP par identification d'un point ou le taux de vrais positifs croise le taux de vrais négatifs.

10. Procédé selon la revendication 8, dans lequel :

(i) ladite sélection de la valeur seuil à partir des niveaux d'analytes combinés d'une manière qui augmente la sensibilité de discrimination entre la BPH et le CaP comprend l'utilisation d'une procédure d'optimisation de Nelder-Mead à somme pondérée pour optimiser pour une aire sous la courbe partielle générée par une analyse ROC (pAUC), dans lequel pAUC représente l'aire sous la ROC dans une plage restreinte (1-sensibilité) qui se trouve entre la valeur 0 et une valeur e spécifiée (par exemple 0,5) ; et/ou
(ii) ladite sélection de la valeur seuil à partir des niveaux d'analytes combinés d'une manière qui augmente la sensibilité de discrimination entre la BPH et le CaP maximise ladite sensibilité, ou minimise ladite spécificité.

11. Procédé selon la revendication 8 ou la revendication 10, dans lequel ladite sélection de la valeur seuil à partir des niveaux d'analytes combinés d'une manière qui augmente la spécificité de discrimination entre la BPH et le CaP comprend l'utilisation d'une procédure d'optimisation de Nelder-Mead à somme pondérée pour optimiser pour une aire sous la courbe partielle générée par une analyse ROC (pAUC), dans lequel pAUC représente l'aire sous la ROC dans une plage restreinte (1-spécificité) qui se trouve entre la valeur 0 et une valeur e spécifiée (par exemple 0,5).

12. Procédé selon l'une quelconque des revendications 8, 10 ou 11, dans lequel les au moins deux analytes sont sélectionnés parmi l'une quelconque des combinaisons d'analytes suivantes :

GPC-1/EGF/follistatine ;
GPC-1/EGF/TNF$\alpha$ ;
GPC-1/HGF ;
GPC-1/HGF/PAI-1 ;
GPC-1/HGF/HuFGFb ;
GPC-1/HGF/EGF ;
GPC-1/HGF/HuPDFGBB ;
GPC-1/EGF ;
GPC-1/PAI-1 ;
GPC-1/G-CSF ; et
GPC-1/PDGFAB.BB.

13. Procédé selon l'une quelconque des revendications 1 à 12, dans lequel les au moins deux analytes sont sélectionnés parmi l'une quelconque des combinaisons d'analytes suivantes :

GPC-1/HGF ;
GPC-1/PAI-1 ;
GPC-1/G-CSF ;
GPC-1/EGF ;
GPC-1/PDGFAB.BB ;
GPC-1/HGF/FGFb ;
GPC-1/HGF/PAI-1 ;
GPC-1/HGF/EGF ;
GPC-1/HGF/follistatine ; et
GPC-1/HGF /G-CSF.

14. Procédé selon l'une quelconque des revendications 1 à 13, dans lequel ladite détection d'au moins deux analytes dans l'échantillon biologique du sujet comprend :

(i) la mesure d'un ou plusieurs signaux de fluorescence représentatifs de chacun desdits niveaux d'analytes ;
(ii) l'obtention d'une mesure de poids/volume desdits analytes dans les échantillons ;
(iii) la mesure d'un signal d'absorbance représentatif de chacun desdits niveaux d'analytes ; ou
(iv) l'utilisation d'une technique sélectionnée dans le groupe consistant en : la spectrométrie de masse, une technique de puce à protéines, la chromatographie en phase liquide haute performance (CLHP), l'électrophorèse sur gel, le radiomarquage, et toute combinaison de ceux-ci.

15. Procédé selon l'une quelconque des revendications 1 à 14, dans lequel :

(i) ladite obtention d'un niveau d'analyte dans les échantillons biologiques de la série et l'échantillon biologique du sujet comprend la mise en contact de chacun desdits échantillons avec des première et seconde populations d'anticorps, dans lequel chacune desdites populations d'anticorps a une spécificité de liaison pour l'un desdits analytes, et les première et seconde populations d'anticorps ont différentes spécificités de liaison ; et/ou
(ii) les échantillons biologiques de la série et l'échantillon biologique du sujet sont chacun du sang total, du sérum, du plasma, de la salive, des larmes, de l'urine, ou un tissu.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- AU 2015902919

### Non-patent literature cited in the description

- SEER Cancer Statistics Factsheets: Prostate Cancer. National Cancer Institute
- **HOFFMAN et al.** *BMC Fam Pract.,* 2002, vol. 3, 19
- **GAO et al.** *Prostate,* 1997, vol. 31, 264-281
- **SHORE et al.** *Journal of Urology,* 17 May 2015, vol. 193 (4), e496-e496
- 2015 Annual Report on Prostate Diseases. Harvard Health Publications, 2015
- **KOHLER ; MILSTEIN.** *Nature,* 1975, vol. 256, 495-497
- **COLIGAN et al.** Methods In Molecular Biology. Humana Press, 1992
- **HARLOW ; LANE.** Antibodies: A Laboratory Manual. Cold Spring Harbor Pub, 1988, 726
- **COLE et al.** Monoclonal Antibodies and Cancer Therapy. Alan R. Liss, Inc, 1985, 77-96
- **KOZBOR et al.** *Immunology Today,* 1983, vol. 4, 72
- The Immunoassay Handbook. Theory and applications of ligand binding, ELISA and related techniques. Elsevier, 2013
- **HARLOW ; LANE.** Using antibodies. A Laboratory Manual. Cold Spring Harbor Laboratory Press, 1999
- **BOLD ; MAHONEY.** *Analytical Biochemistry,* 1997, vol. 257, 185-192
- **BREEN et al.** *Cytokine,* 2015, vol. 71, 188-198
- **ROBIN et al.** *BMC Bioinformatics,* 2011, vol. 12, 77
- **PEPE et al.** *Biometrics,* 2003, vol. 59, 133